# EUROPEAN PATENT APPLICATION

(11) **EP 4 032 906 A1**
(43) Date of publication of application: **27.07.2022**
(21) Application number: 21217955.0
(22) Date of filing: 11.10.2017
(51) Int. Cl.: C07K 16/24, A61K 39/395, A61P 29/00

(54) **METHODS OF TREATING DISEASES**

(30) Priority: 14.10.2016 US 201662408127 P
(62) Divisional of application: 20215472.0
(71) Applicant: Boehringer Ingelheim International GmbH, 55216 Ingelheim am Rhein (DE)
(72) Inventor: Baum, Patrick, 55216 Ingelheim am Rhein (DE); Boecher, Wulf Otto, 55216 Ingelheim am Rhein (DE); Galler, Annette Bettina, 55216 Ingelheim am Rhein (DE); Lalovic, Bojan, Ridgefield, CT 06877-0368 (US); Visvanathan, Sudha, Ridgefield, CT 06877-0368 (US)
(74) Representative: Hoffmann Eitle

(57) **Abstract**

This invention generally relates to methods for the treatment of IL-23 related diseases, in particular inflammatory diseases, such as Crohn's Disease, utilizing anti-IL-23A antibodies.

SEQUENCE LISTING

<110> BOEHRINGER INGELHEIM INTERNATIONAL GMBH

<120> METHODS OF TREATING DISEASES

<130> 114758-0137

<140> PCT/US2017/056082
<141> 2017-10-11

<150> 62/408,127
<151> 2016-10-14

<160> 22

<170> PatentIn version 3.5

<210> 1
<211> 11
<212> PRT
<213> Artificial Sequence

<220>
<221> source
<223> /note="Description of Artificial Sequence: Synthetic
      peptide"

<400> 1
Lys Ala Ser Arg Asp Val Ala Ile Ala Val Ala
1               5                   10


<210> 2
<211> 7
<212> PRT
<213> Artificial Sequence

<220>
<221> source
<223> /note="Description of Artificial Sequence: Synthetic
      peptide"

<400> 2
Trp Ala Ser Thr Arg His Thr
1               5


<210> 3
<211> 9
<212> PRT
<213> Artificial Sequence

<220>
<221> source
<223> /note="Description of Artificial Sequence: Synthetic
      peptide"

<400> 3
His Gln Tyr Ser Ser Tyr Pro Phe Thr
1               5

<210> 4
<211> 10
<212> PRT
<213> Artificial Sequence

<220>
<221> source
<223> /note="Description of Artificial Sequence: Synthetic
      peptide"

<400> 4
Gly Asn Thr Phe Thr Asp Gln Thr Ile His
1               5                   10


<210> 5
<211> 17
<212> PRT
<213> Artificial Sequence

<220>
<221> source
<223> /note="Description of Artificial Sequence: Synthetic
      peptide"

<400> 5
Tyr Ile Tyr Pro Arg Asp Asp Ser Pro Lys Tyr Asn Glu Asn Phe Lys
1               5                   10                  15


Gly




<210> 6
<211> 11
<212> PRT
<213> Artificial Sequence

<220>
<221> source
<223> /note="Description of Artificial Sequence: Synthetic
      peptide"

<400> 6
Pro Asp Arg Ser Gly Tyr Ala Trp Phe Ile Tyr
1               5                   10


<210> 7
<211> 10
<212> PRT
<213> Artificial Sequence

<220>
<221> source
<223> /note="Description of Artificial Sequence: Synthetic
      peptide"

<400> 7
Gly Tyr Thr Phe Thr Asp Gln Thr Ile His
1               5                   10

<210> 8
<211> 10
<212> PRT
<213> Artificial Sequence

<220>
<221> source
<223> /note="Description of Artificial Sequence: Synthetic
      peptide"

<400> 8
Gly Phe Thr Phe Thr Asp Gln Thr Ile His
1               5                   10


<210> 9
<211> 10
<212> PRT
<213> Artificial Sequence

<220>
<221> source
<223> /note="Description of Artificial Sequence: Synthetic
      peptide"

<400> 9
Gly Gly Thr Phe Thr Asp Gln Thr Ile His
1               5                   10


<210> 10
<211> 107
<212> PRT
<213> Artificial Sequence

<220>
<221> source
<223> /note="Description of Artificial Sequence: Synthetic
      polypeptide"

<400> 10
Asp Ile Gln Met Thr Gln Ser Pro Ser Ser Leu Ser Ala Ser Val Gly
1               5                   10                  15


Asp Arg Val Thr Ile Thr Cys Lys Ala Ser Arg Asp Val Ala Ile Ala
            20                  25                  30


Val Ala Trp Tyr Gln Gln Lys Pro Gly Lys Val Pro Lys Leu Leu Leu
        35                  40                  45


Phe Trp Ala Ser Thr Arg His Thr Gly Val Pro Asp Arg Phe Ser Gly
    50                  55                  60


Ser Gly Ser Gly Thr Asp Phe Thr Leu Thr Ile Ser Ser Leu Gln Pro
65                  70                  75                  80


Glu Asp Leu Ala Asp Tyr Tyr Cys His Gln Tyr Ser Ser Tyr Pro Phe

85                  90                  95

Thr Phe Gly Gln Gly Thr Lys Leu Glu Ile Lys
            100                 105

<210> 11
<211> 107
<212> PRT
<213> Artificial Sequence

<220>
<221> source
<223> /note="Description of Artificial Sequence: Synthetic
      polypeptide"

<400> 11
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Ile
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Asp Tyr Phe Cys His Gln Tyr Ser Ser Tyr Pro Phe
                85                  90                  95

Thr Phe Gly Ser Gly Thr Lys Leu Glu Ile Lys
            100                 105

<210> 12
<211> 107
<212> PRT
<213> Artificial Sequence

<220>
<221> source
<223> /note="Description of Artificial Sequence: Synthetic
      polypeptide"

<400> 12
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Thr Tyr Tyr Cys His Gln Tyr Ser Ser Tyr Pro Phe
                85                  90                  95

Thr Phe Gly Ser Gly Thr Lys Leu Glu Ile Lys
            100                 105

<210> 13
<211> 107
<212> PRT
<213> Artificial Sequence

<220>
<221> source
<223> /note="Description of Artificial Sequence: Synthetic
      polypeptide"

<400> 13
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Arg Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Ser Gly Thr Lys Leu Glu Ile Lys
            100                 105

<210> 14
<211> 120

<212> PRT
<213> Artificial Sequence

<220>
<221> source
<223> /note="Description of Artificial Sequence: Synthetic
      polypeptide"

<400> 14
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Gln
            20                  25                  30

Thr Ile His Trp Met Arg Gln Ala Pro Gly Gln Gly Leu Glu Trp Ile
        35                  40                  45

Gly Tyr Ile Tyr Pro Arg Asp Asp Ser Pro Lys Tyr Asn Glu Asn Phe
    50                  55                  60

Lys Gly Lys Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120


<210> 15
<211> 120
<212> PRT
<213> Artificial Sequence

<220>
<221> source
<223> /note="Description of Artificial Sequence: Synthetic
      polypeptide"

<400> 15
Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Lys Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120

<210> 16
<211> 120
<212> PRT
<213> Artificial Sequence

<220>
<221> source
<223> /note="Description of Artificial Sequence: Synthetic
      polypeptide"

<400> 16
Gln Val Gln Leu Val Gln Ser Gly Ala Glu Val Lys Lys Pro Gly Ser
1               5                   10                  15

Ser Val Lys Thr Ser Cys Lys Ala Ser Gly Gly Thr Phe Thr Asp Gln
            20                  25                  30

Thr Ile His Trp Val Arg Gln Arg Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser
        115                 120

<210> 17
<211> 120
<212> PRT
<213> Artificial Sequence

<220>
<221> source
<223> /note="Description of Artificial Sequence: Synthetic
      polypeptide"

<400> 17
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Pro Asp Arg Ser Gly Tyr Ala Trp Phe Ile Tyr Trp Gly Gln
            100                 105                 110

Gly Thr Leu Val Thr Val Ser Ser
        115                 120


<210> 18
<211> 214
<212> PRT
<213> Artificial Sequence

<220>
<221> source
<223> /note="Description of Artificial Sequence: Synthetic
      polypeptide"

<400> 18
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Ile
        35                  40                  45

Tyr Trp Ala Ser Thr Arg His Thr Gly Val Pro Ser Arg Phe Ser Gly
    50                  55                  60

Ser Gly Ser Arg Thr Asp Phe Thr Leu Thr Ile Ser Ser Leu Gln Pro
65                  70                  75                  80

Glu Asp Val Ala Asp Tyr Phe Cys His Gln Tyr Ser Ser Tyr Pro Phe
                85                  90                  95

Thr Phe Gly Ser Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 19
<211> 449
<212> PRT
<213> Artificial Sequence

<220>
<221> source
<223> /note="Description of Artificial Sequence: Synthetic
      polypeptide"

<400> 19
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Gln
            20                  25                  30

Thr Ile His Trp Met Arg Gln Ala Pro Gly Gln Gly Leu Glu Trp Ile
        35                  40                  45

Gly Tyr Ile Tyr Pro Arg Asp Asp Ser Pro Lys Tyr Asn Glu Asn Phe
    50                  55                  60

Lys Gly Lys Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 20
<211> 449
<212> PRT
<213> Artificial Sequence

<220>
<221> source
<223> /note="Description of Artificial Sequence: Synthetic
      polypeptide"

<400> 20
Gln Val Gln Leu Val Gln Ser Gly Ala Glu Val Lys Lys Pro Gly Ser
1               5                   10                  15

Ser Val Lys Val Ser Cys Lys Ala Ser Gly Phe Thr Phe Thr Asp Gln
            20                  25                  30

Thr Ile His Trp Val Arg Gln Ala Pro Gly Gln Gly Leu Glu Trp Met
        35                  40                  45

Gly Tyr Ile Tyr Pro Arg Asp Asp Ser Pro Lys Tyr Asn Glu Asn Phe
    50                  55                  60

Lys Gly Lys Val Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ile Pro Asp Arg Ser Gly Tyr Ala Trp Phe Ile Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 21
<211> 214
<212> PRT
<213> Artificial Sequence

<220>
<221> source
<223> /note="Description of Artificial Sequence: Synthetic
      polypeptide"

<400> 21
Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asp Val Ala Ile Ala
            20                  25                  30

Val Ala Trp Tyr Gln Gln Lys Pro Gly Lys Val Pro Lys Leu Leu Leu
        35                  40                  45

Phe Trp Ala Ser Thr Arg His Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Leu Ala Asp Tyr Tyr Cys His Gln Tyr Ser Ser Tyr Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 22
<211> 189
<212> PRT
<213> Homo sapiens

<400> 22
Met Leu Gly Ser Arg Ala Val Met Leu Leu Leu Leu Leu Pro Trp Thr
1               5                   10                  15

Ala Gln Gly Arg Ala Val Pro Gly Gly Ser Ser Pro Ala Trp Thr Gln
            20                  25                  30

Cys Gln Gln Leu Ser Gln Lys Leu Cys Thr Leu Ala Trp Ser Ala His
        35                  40                  45

Pro Leu Val Gly His Met Asp Leu Arg Glu Glu Gly Asp Glu Glu Thr
    50                  55                  60

Thr Asn Asp Val Pro His Ile Gln Cys Gly Asp Gly Cys Asp Pro Gln
65                  70                  75                  80

Gly Leu Arg Asp Asn Ser Gln Phe Cys Leu Gln Arg Ile His Gln Gly
                85                  90                  95

Leu Ile Phe Tyr Glu Lys Leu Leu Gly Ser Asp Ile Phe Thr Gly Glu
            100                 105                 110

Pro Ser Leu Leu Pro Asp Ser Pro Val Gly Gln Leu His Ala Ser Leu
        115                 120                 125

Leu Gly Leu Ser Gln Leu Leu Gln Pro Glu Gly His His Trp Glu Thr
    130                 135                 140

Gln Gln Ile Pro Ser Leu Ser Pro Ser Gln Pro Trp Gln Arg Leu Leu
145                 150                 155                 160

Leu Arg Phe Lys Ile Leu Arg Ser Leu Gln Ala Phe Val Ala Val Ala
                165                 170                 175

Ala Arg Val Phe Ala His Gly Ala Ala Thr Leu Ser Pro
            180                 185

## Description

### Cross Reference to Related Applications

This application claims priority from U.S. Provisional Application No. 62/408,127, filed October 14, 2016, the entire contents of which are incorporated herein by reference.

### Technical Field of the Invention

This invention generally relates to method of treating inflammatory diseases, for example Crohn's Disease (CD), utilizing anti-IL-23A antibodies.

### Background of the Invention

Crohn's Disease (CD) is a chronic relapsing, remitting inflammatory disease of the gastrointestinal tract characterized by abdominal pain, fever, and bloody or mucus-containing diarrhea. The disease affects the gastrointestinal tract discontinuously from mouth to anus, but most commonly the ileum and colon (40%), followed by the small bowel only (30%), and the colon only (25%). It occurs in a relatively young population and there is no marked sex difference.

The incidence of CD seems to be increasing with more recent estimates varying from 7.9 to 20.2 cases/100,000, and a prevalence of 161 to 319 cases/100,000 in North America and Europe. Mucosal lesions may be complicated by perforation and fistula formation, which may require hospitalization for medical or surgical management.

There is a need for treatment options for inflammatory diseases, in particular Crohn's Disease, that lead to favorable outcomes for patients, for example in terms of efficacy, safety and/or tolerability of the treatment.

### Summary of the Invention

The present invention addresses the above needs and provides methods for treating inflammatory diseases, in particular methods comprising administering an anti-IL-23A antibody to a patient in certain amounts and/or at certain intervals. In one aspect, a method of the present invention is for the treament of Crohn's Disease. In one aspect, a method of the present invention is for the treament of ulcerative colitis.

The methods of the present invention provide the advantage of enabling patients to experience clinical improvement while receiving fewer administrations of the anti-IL-23A antibody.

In one embodiment, the present invention provides a method for treating an inflammatory disease, in one aspect for treating Crohn's Disease, comprising (a) administering to a patient a dose of an anti-IL-23A antibody at week 0, at week 4 and at week 8 by intravenous infusion, wherein the doses of the anti-IL-23A antibody comprise 200 mg or 600 mg of the antibody. In one embodiment, the method further comprises (b) administering to the patient three doses of the anti-IL-23A antibody, for example at week 14, at week 18 and at week 22 by intravenous infusion, wherein the doses of the anti-IL-23A antibody comprise 600 mg of the antibody. In one embodiment, the method further comprises (c) administering to the patient one or more doses of the anti-IL-23A antibody at 8 weeks intervals by subcutaneous injection, for example four doses of the anti-IL-23A antibody at 8 weeks intervals by subcutaneous injection, for example at week 26, at week 34, at week 42 and at week 50, wherein the one or more doses of the anti-IL-23A antibody comprise 180 mg of the antibody.

In one embodiment, at week 12, a patient is evaluated for deep remission, for example defined as reaching clinical remission (CDAI score <150) and endoscopic remission (CDEIS ≤ 4). In one, aspect, for a patient with initial isolated ileitis endoscopic remission is defined by CDEIS ≤ 2.

In one embodiment, the present invention provides a method for treating an inflammatory disease, in one aspect for treating Crohn's Disease, comprising (a) administering to a patient a dose of an anti-IL-23A antibody at week 0, at week 4 and at week 8 by intravenous infusion, wherein the doses of the anti-IL-23A antibody comprise 200 mg or 600 mg of the antibody. In one embodiment, the method further comprises (b) administering to the patient one or more doses of the anti-IL-23A antibody at 8 weeks intervals by subcutaneous injection, for example four doses of the anti-IL-23A antibody at 8 weeks intervals by subcutaneous injection, for example at week 26, at week 34, at week 42 and at week 50, wherein the one or more doses of the anti-IL-23A antibody comprise 180 mg of the antibody.

In one embodiment, at week 12, a patient is evaluated for deep remission, for example defined as reaching clinical remission (CDAI score <150) and endoscopic remission (CDEIS ≤ 4). In one, aspect, for a patient with initial isolated ileitis endoscopic remission is defined by CDEIS ≤ 2.

In one embodiment, the present invention provides a method for treating an inflammatory disease, in one aspect for treating Crohn's Disease, comprising administering to a patient 180 mg of the anti-IL-23A antibody at 8 weeks intervals by subcutaneous injection.

In one embodiment, the present invention provides a method for treating an inflammatory disease, in one aspect for treating Crohn's Disease, comprising administering to a patient an anti-IL-23A antibody, said method comprising administering at least one induction dose of the anti-IL-23A antibody to the patient, wherein said induction dose comprises 200 to 1,200 mg of the anti-IL-23A antibody, for example 450 to 1,200 mg of the anti-IL-23A antibody. In one aspect, the induction dose comprises 200 mg, 450 mg, 600 mg, 900 mg or 1,200 mg of the anti-IL-23A antibody. In one aspect, the induction dose comprises 750 mg of the anti-IL-23A antibody. In one aspect, 1, 2 or 3 induction doses are administered to the patient. In one aspect, 2 or 3 inductions doses are administered, for example at 4 weeks intervals. In one aspect, the induction dose(s) is administered by intravenous infusion.

In one embodiment, the induction doses comprise 200 mg of the anti-IL-23A antibody and three inductions doses are administered to the patient at 4 weeks intervals.

In one embodiment, the induction doses comprise 450 mg of the anti-IL-23A antibody and three inductions doses are administered to the patient at 4 weeks intervals.

In one embodiment, the induction doses comprise 600 mg of the anti-IL-23A antibody and three inductions doses are administered to the patient at 4 weeks intervals.

In one embodiment, the induction doses comprise 900 mg of the anti-IL-23A antibody and three inductions doses are administered to the patient at 4 weeks intervals.

In one embodiment, the induction doses comprise 1,200 mg of the anti-IL-23A antibody and three inductions doses are administered to the patient at 4 weeks intervals.

In one embodiment, the induction doses comprise 750 mg of the anti-IL-23A antibody and three inductions doses are administered to the patient at 4 weeks intervals.

In one embodiment, at least one additional induction dose of the anti-IL-23A antibody is administered to the patient after the last induction dose above. In one aspect, an additional induction dose comprises 200 to 1,200 mg of the anti-IL-23A antibody, for example 450 to 1,200 mg of the anti-IL-23A antibody. In one aspect, an additional induction dose comprises 200 mg, 450 mg, 600 mg, 900 mg or 1,200 mg of the anti-IL-23A antibody. In one aspect, an additional induction dose comprises 750 mg of the anti-IL-23A antibody. In one aspect, 1, 2 or 3 additional induction doses are administered to the patient. In one aspect, 2 or 3 additional inductions doses are administered, for example at 4 weeks intervals. In one aspect, the additional induction dose(s) is administered by intravenous infusion.

In one embodiment, the patient has a CDAI score of 220-450 before the administration of the first induction dose.

In one embodiment, the patient achieves clinical remission after the administration of the one or more induction doses. In one embodiment, the patient achieves a CDAI score of less than 150 after the administration of the one or more induction doses. In one embodiment, the patient achieves a PRO-2 score equal to or less than 75 after the administration of the one or more induction doses. In one embodiment, the patient achieves a CDAI score of less than 150 and a PRO-2 score equal to or less than 75 after the administration of the one or more induction doses.

In one embodiment, the present invention further provides a method for inducing clinical remission of Crohn's Disease in a patient comprising administering to the patient an anti-IL-23 antibody as described above or herein.

In one embodiment, the present invention further provides a method for inducing clinical response to Crohn's Disease in a patient comprising administering to the patient an anti-IL-23 antibody as described above or herein.

In one ambodiment, the method further comprises administering a first maintenance dose of the anti-IL-23A antibody to the patient after the last induction dose is administered and administering at least one additional maintenance dose to the patient 4 to 12 weeks after said first maintenance dose is administered. In one aspect, the first maintenance dose is administered 2 to 8 weeks, for example 4 to 6 weeks, for example 2 weeks, 4 weeks, 6 weeks or 8 weeks, after the last induction dose is administered. In one aspect, the at least one additional maintenance dose is administered to the patient 4, 8 or 12 weeks after the first maintenance dose is administered.

In one embodiment, the first maintenance dose comprises 150 to 300 mg of the anti-IL-23A antibody. In one aspect, the first maintenance dose comprises 150 mg, 225 mg or 300 mg of the anti-IL-23A antibody. In one aspect, the first maintenance dose comprises 180 mg or 270 mg of the anti-IL-23A antibody.

In one aspect, the at least one additional maintenance dose comprises 150 to 300 mg of the anti-IL-23A antibody. In one aspect, the at least one additional maintenance dose comprises 150 mg, 225 mg or 300 mg of the anti-IL-23A antibody. In one aspect, the at least one additional maintenance dose comprises 180 mg or 270 mg of the anti-IL-23A antibody.

In one aspect, the first maintenance dose and the at least one additional maintenance dose comprise 150 to 300 mg of the anti-IL-23A antibody. In one aspect, the first maintenance dose and the at least one additional maintenance dose comprise 150 mg, 225 mg or 300 mg of said anti-IL-23A antibody. In one aspect, the first maintenance dose and the at least one additional maintenance dose comprise 180 mg or 270 mg of said anti-IL-23A antibody.

In one aspect, a maintenance dose is administered by subcutaneous injection.

In one embodiment, the maintenance doses comprise 150 mg of the anti-IL-23A antibody and are administered to the patient at 4 weeks intervals.

In one embodiment, the maintenance doses comprise 150 mg of the anti-IL-23A antibody and are administered to the patient at 8 weeks intervals.

In one embodiment, the maintenance doses comprise 225 mg of the anti-IL-23A antibody and are administered to the patient at 8 weeks intervals.

In one embodiment, the maintenance doses comprise 225 mg of the anti-IL-23A antibody and are administered to the patient at 12 weeks intervals.

In one embodiment, the maintenance doses comprise 300 mg of the anti-IL-23A antibody and are administered to the patient at 8 weeks intervals.

In one embodiment, the maintenance doses comprise 300 mg of the anti-IL-23A antibody and are administered to the patient at 12 weeks intervals.

In one embodiment, the patient maintains clinical remission after the administration of the one or more maintenance doses. In one embodiment, the patient maintains a CDAI score of less than 150 after the administration of the one or more maintenance doses. In one embodiment, the patient maintains a PRO-2 score equal to or less than 75 after the administration of the one or more maintenance doses. In one embodiment, the patient maintains a CDAI score of less than 150 and a PRO-2 score equal to or less than 75 after the administration of the one or more maintenance doses.

In one embodiment, the present invention further provides a method for maintaining clinical remission of Crohn's Disease in a patient comprising administering to the patient an anti-IL-23 antibody as described above or herein.

In one embodiment, the present invention further provides a method for maintaining clinical response to Crohn's Disease in a patient comprising administering to the patient an anti-IL-23 antibody as described above or herein.

In one embodiment, the present invention further provides a method for treating Crohn's Disease by inducing and maintaining clinical remission in a patient comprising administering to the patient an anti-IL-23 antibody as described above or herein.

In one embodiment, the present invention further provides a method for maintaining endoscopic remission of Crohn's Disease in a patient comprising administering to the patient an anti-IL-23 antibody as described above or herein.

In one embodiment,the present invention provides a method for inducing clinical remission of Crohn's Disease comprising administering to a patient an anti-IL-23A antibody, said method comprising administering at least one induction dose of said anti-IL-23A antibody to the patient, wherein said induction dose comprises 200 to 1,200 mg of said anti-IL-23A antibody. In one embbodiment, the induction dose comprises 450 to 1,200 mg of said anti-IL-23A antibody. In one embodiment, the induction dose comprises 200 mg, 450 mg, 600 mg, 900 mg or 1,200 mg of said anti-IL-23A antibody. In one embodiment, the induction dose comprises 750 mg of said anti-IL-23A antibody. In one embodiment, 1, 2 or 3 induction doses are administered to the patient. In one embodiment, 2 or 3 inductions doses are administered at 4 weeks intervals. In one embodiment, the induction dose(s) is/are administered by intravenous infusion. In one embodiment, the patient has a CDAI score of 220-450 before said administration. In one embodiment, the patient achieves a CDAI score of less than 150. In one embodiment, the patient achieves a PRO-2 score equal to or less than 75.

In one embodiment, the method further comprises maintaining clinical remission of Crohn's disease, said method further comprising administering a first maintenance dose of said anti-IL-23A antibody to the patient after the last induction dose is administered, and administering at least one additional maintenance dose to the patient 4 to 12 weeks after said first maintenance dose is administered. In one embodiment, the first maintenance dose is administered 2 to 8 weeks, for example 4 to 6 weeks, for example 2 weeks, 4 weeks, 6 weeks or 8 weeks, after the last induction dose is administered. In one embodiment, the at least one additional maintenance dose is administered to the patient 4, 8 or 12 weeks after after said first maintenance dose is administered. In one embodiment, the first maintenance dose comprises 150 to 300 mg of said anti-IL-23A antibody. In one embodiment, the first maintenance dose comprises 150 mg, 225 mg or 300 mg of said anti-IL-23A antibody. In one embodiment, the first maintenance dose comprises 180 mg or 270 mg of said anti-IL-23A antibody. In one embodiment, the at least one additional maintenance dose comprises 150 to 300 mg of said anti-IL-23A antibody. In one embodiment, the at least one additional maintenance dose comprises 150 mg, 225 mg or 300 mg of said anti-IL-23A antibody. In one embodiment, the at least one additional maintenance dose comprises 180 mg or 270 mg of said anti-IL-23A antibody. In one embodiment, the first maintenance dose and said at least one additional maintenance dose comprise 150 to 300 mg of said anti-IL-23A antibody. In one embodiment, the first maintenance dose and said at least one additional maintenance dose comprise 150 mg, 225 mg or 300 mg of said anti-IL-23A antibody. In one embodiment, the first maintenance dose and said at least one additional maintenance dose comprise 180 mg or 270 mg of said anti-IL-23A antibody. In one embodiment, the maintenance dose is administered by subcutaneous injection. In one embodiment, the patient maintains a CDAI score of less than 150. In one embodiment, the patient maintains a PRO-2 score equal to or less than 75.

In one embodiment, the present invention provides a method for treating an inflammatory disease, in one aspect for treating Crohn's Disease, comprising administering to a patient 150 to 1,200 mg of an anti-IL-23A antibody. In one aspect, the method comprises administering to a patient 200 mg to 1,200 mg of an anti-IL-23A antibody, for example 450 to 1,200 mg of an anti-IL-23A antibody. In one aspect, the method comprises administering to a patient 200 mg, 450 mg, 600 mg, 900 mg or 1,200 mg of an anti-IL-23A antibody. In one aspect, the method comprises administering to a patient 750 mg of an anti-IL-23A antibody. In one aspect, the method comprises administering to a patient 150 to 300 mg of an anti-IL-23A antibody. In one aspect, the method comprises administering to a patient 150 mg, 225 mg or 300 mg of an anti-IL-23A antibody. In one aspect, the method comprises administering to a patient 180 mg or 270 mg of an anti-IL-23A antibody.

In one embodiment, in any one of the methods above, the anti-IL-23A antibody is Antibody A, Antibody B, Antibody C or Antibody D.

In one embodiment, in any one of the methods above, the method is for treating Crohn's Disease, for example moderately to severely active Crohn's Disease. In one aspect, in the context of a method of the present invention a patient is naive to, or was previously treated with anti-TNF therapy. In one aspect, in the context of a method of the present invention a patient was previously treated with one, two, three or more TNF antagonist(s). In one embodiment, the patient is a patient who had an inadequate response with, was intolerant to, or demonstrated dependence on corticosteroids. In one embodiment, the patient is a patient who had an inadequate response with, lost response, or was intolerant to an immunomodulator, a TNFα inhibitor (or TNF antagonist) or an integrin inhibitor. In one embodiment, the treatment is by inducing and maintaining clinical remission, corticosteroid-free remission, endoscopic remission and mucosal healing.

In one embodiment, a patient to be treated by a method according ot the present invention has a CDAI score of 220-450.

In one embodiment, in any one of the methods above, the patient is an adult patient.

In one embodiment, in any one of the methods above, the method is for treating ulcerative colitis.

In one aspect, the present invention provides an anti-IL-23A antibody for use in the treament of a disease, for example an inflammatory disease, for example Crohn's Disease, by administration in certain amounts and/or at certain intervals as described herein. In one aspect, the inflammatory disease is ulcerative colitis.

In one aspect, the present invention provides for the use of an anti-IL-23A antibody for the preparation of a medicament for the treatment of a disease, for example an inflammatory disease, for example Crohn's Disease, by administration in certain amounts and/or at certain intervals as described herein. In one aspect, the inflammatory disease is ulcerative colitis.

In one embodiment, in any one of the methods or uses above, the anti-IL-23A antibody is disclosed below.

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO: 4, 7, 8 or 9 *(CDR1-H);* the amino acid sequence of SEQ ID NO:5 *(CDR2-H*); and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:4 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:7 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:8 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of SEQ ID NO:1 *(CDR1-L);* the amino acid sequence of SEQ ID NO:2 *(CDR2-L);* and the amino acid sequence of SEQ ID NO:3 *(CDR3-L);* and a heavy chain variable region comprising the amino acid sequence of SEQ ID NO:9 *(CDR1-H);* the amino acid sequence of SEQ ID NO:5 *(CDR2-H);* and the amino acid sequence of SEQ ID NO:6 *(CDR3-H).*

In one embodiment, the anti-IL-23A antibody comprises a light chain variable region comprising the amino acid sequence of any one of SEQ ID NO:10, 11, 12 or 13; and a heavy chain variable region comprising the amino acid sequence any one of SEQ ID NO:14, 15, 16 or 17.

In one embodiment, the anti-IL-23A antibody comprises a light chain variable region comprising the amino acid sequence of SEQ ID NO:11 and a heavy chain variable region comprising the amino acid sequence SEQ ID NO:14.

In one embodiment, the anti-IL-23A antibody comprises a light chain variable region comprising the amino acid sequence of SEQ ID NO:11 and a heavy chain variable region comprising the amino acid sequence SEQ ID NO:15.

In one embodiment, the anti-IL-23A antibody comprises a light chain variable region comprising the amino acid sequence of SEQ ID NO:10 and a heavy chain variable region comprising the amino acid sequence SEQ ID NO:14.

In one embodiment, the anti-IL-23A antibody comprises a light chain variable region comprising the amino acid sequence of SEQ ID NO:10 and a heavy chain variable region comprising the amino acid sequence SEQ ID NO:15.

In one embodiment, the anti-IL-23A antibody comprises the amino acid sequence SEQ ID NO:14 or 15 linked to a human IgG1, IgG2, IgG3, IgG4, IgM, IgA or IgE heavy chain constant region. In one embodiment, the anti-IL-23A antibody comprises the amino acid sequence of SEQ ID NO: 14 or 15 linked to a human IgG1 heavy chain constant region. In one embodiment, the anti-IL-23A antibody comprises the amino acid sequence of SEQ ID NO:10 or 11 linked to a human kappa or lambda light chain constant region.

In one embodiment, In one embodiment, the anti-IL-23A antibody comprises the amino acid sequence of SEQ ID NO:14 or 15 linked to a human IgG1 heavy chain constant region; and the amino acid sequence of SEQ ID NO: 10 or 11 linked to a human kappa light chain constant region.

In one embodiment, the anti-IL-23A antibody is a humanized monoclonal antibody comprising a light chain variable region comprising the amino acid sequence selected from the group consisting of any one of SEQ ID NO:10, 11, 12 and 13 and a heavy chain variable region comprising the amino acid sequence selected from the group consisting of any one of SEQ ID NO:14, 15, 16 and 17.

In one embodiment, the anti-IL-23A antibody is a humanized monoclonal antibody comprising a light chain variable region comprising the amino acid sequence of SEQ ID NO:11 and a heavy chain variable region comprising the amino acid sequence SEQ ID NO:14.

In one embodiment, the anti-IL-23A antibody is a humanized monoclonal antibody comprising a light chain variable region comprising the amino acid sequence of SEQ ID NO:11 and a heavy chain variable region comprising the amino acid sequence SEQ ID NO:15.

In one embodiment, the anti-IL-23A antibody is a humanized monoclonal antibody comprising a light chain variable region comprising the amino acid sequence of SEQ ID NO:10 and a heavy chain variable region comprising the amino acid sequence SEQ ID NO:14.

In one embodiment, the anti-IL-23A antibody is a humanized monoclonal antibody comprising a light chain variable region comprising the amino acid sequence of SEQ ID NO:10 and a heavy chain variable region comprising the amino acid sequence SEQ ID NO:15.

In one embodiment, the anti-IL-23A antibody comprises a light chain comprising the amino acid sequence of SEQ ID NO:18 or 21 and a heavy chain comprising the amino acid sequence of SEQ ID NO:19 or 20.

In one embodiment, the anti-IL-23A antibody comprises a light chain comprising the amino acid sequence of SEQ ID NO:18 and a heavy chain comprising the amino acid sequence of SEQ ID NO:19.

In one embodiment, the anti-IL-23A antibody comprises a light chain comprising the amino acid sequence of SEQ ID NO:18 and a heavy chain comprising the amino acid sequence of SEQ ID NO:20.

In one embodiment, the anti-IL-23A antibody comprises a light chain comprising the amino acid sequence of SEQ ID NO:21 and a heavy chain comprising the amino acid sequence of SEQ ID NO:19.

In one embodiment, the anti-IL-23A antibody comprises a light chain comprising the amino acid sequence of SEQ ID NO: 21 and a heavy chain comprising the amino acid sequence of SEQ ID NO: 20.

In one embodiment, the anti-IL-23A antibody is Antibody A, Antibody B, Antibody C or Antibody D.

In one embodiment, the anti-IL-23A antibody is as disclosed in WO2007/005955, WO2007/024846, WO2007/027714, WO2007/076524, WO2008/103432 or WO2012/061448.

In one embodiment, the present invention provides a method for detecting the presence or absence of a beneficial response in a patient after administration of an IL-23A antagonist, comprising: a) obtaining a biological sample from the patient; b) measuring in said sample the level of expression of one or more genes, proteins or miRNAs; c) comparing the level in b) to the level of expression of the one or more genes, proteins or miRNAs in a control; and d) determining whether or not the difference in levels between the sample and the control reflects a beneficial response in the patient, wherein the one or more genes, proteins or miRNAs is one or more genes, proteins or miRNAs described herein. In one aspect, the patient suffers from Crohns' Disease. In one aspect, the biological sample is a colon tissue or a ileum tissue. In one aspect, the biological sample is taken from the upper gatrointestinal (GI) tract, for example the stomach or the esophagus. In one aspect, the IL-23A antagonist is an anti-IL-23A antibody or an antigen binding fragment thereof, for example Antibody A, Antibody B, Antibody C or Antibody D.

In one embodiment, the anti-IL-23A antibody modulates the genes as described herein. In one embodiment, the anti-IL-23A antibody modulates the pathways as described herein.

In one embodiment, the anti-IL-23A antibody modulates the proteins as described herein, for example faecal calprotectin and/or faecal lactoferrin as described herein.

In one embodiment, the anti-IL-23A antibody modulates miRNAs, for example miR-223-3p, as described herein.

### Brief Description of the Figures

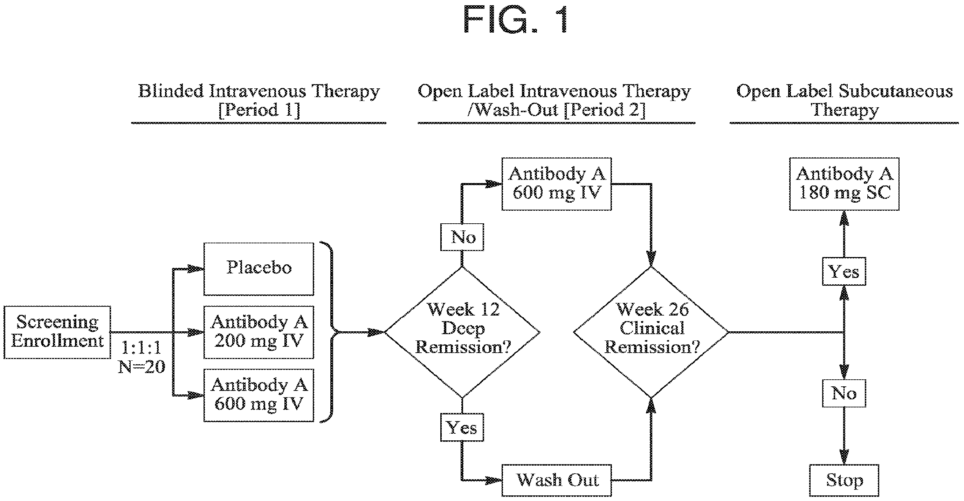

Figure 1: Trial Design. Deep remission, defined as achieving clinical remission (CDAI score < 150) and CDEIS remission (CDEIS score ≤ 4 or 2 or less in patients with initial isolated ileitis).

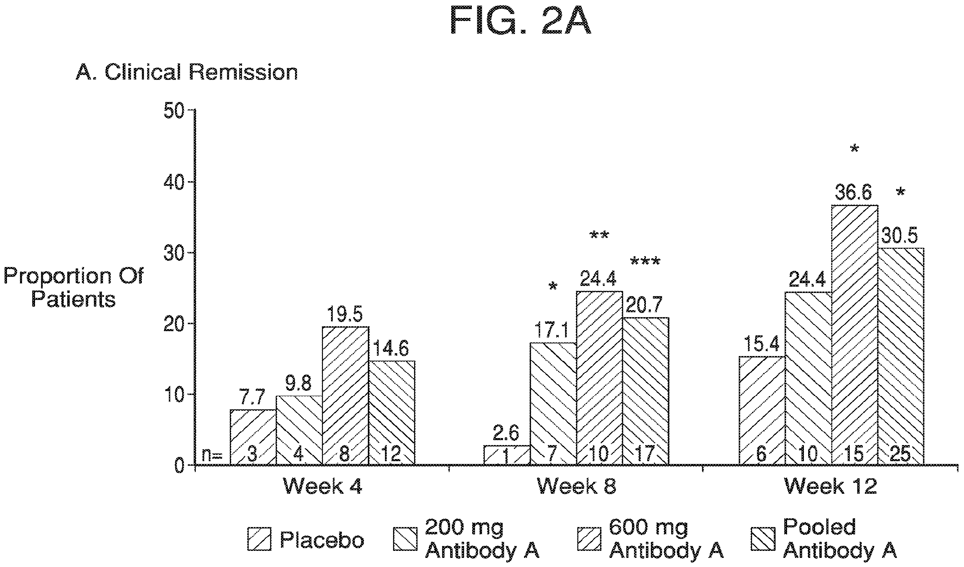

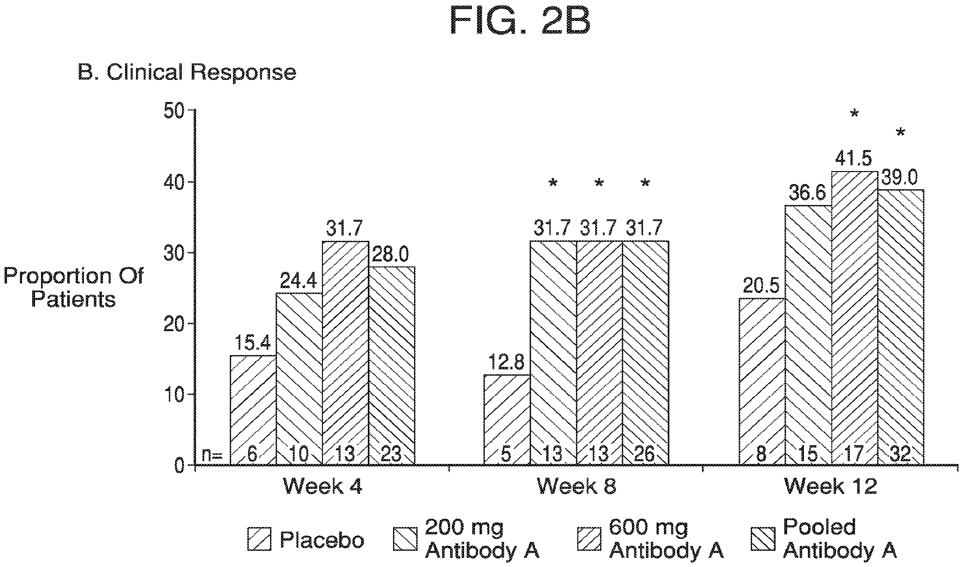

Figure 2A and 2B: Proportion of patients with clinical remission (A) or clinical response (B) at week 4, week 8 and week 12. Clinical remission is defined as a CDAI score <150. Clinical response is either a CDAI score <150 or a CDAI reduction from baseline of ≥100 points. Patients in which prohibited concomitant medication to treat Crohn's disease was used prior to Week 12 were considered as treatment failures. Full analysis set was used for these analyses. *p<0.05 versus placebo, **p<0.005 versus placebo, ***p<0.001 versus placebo.

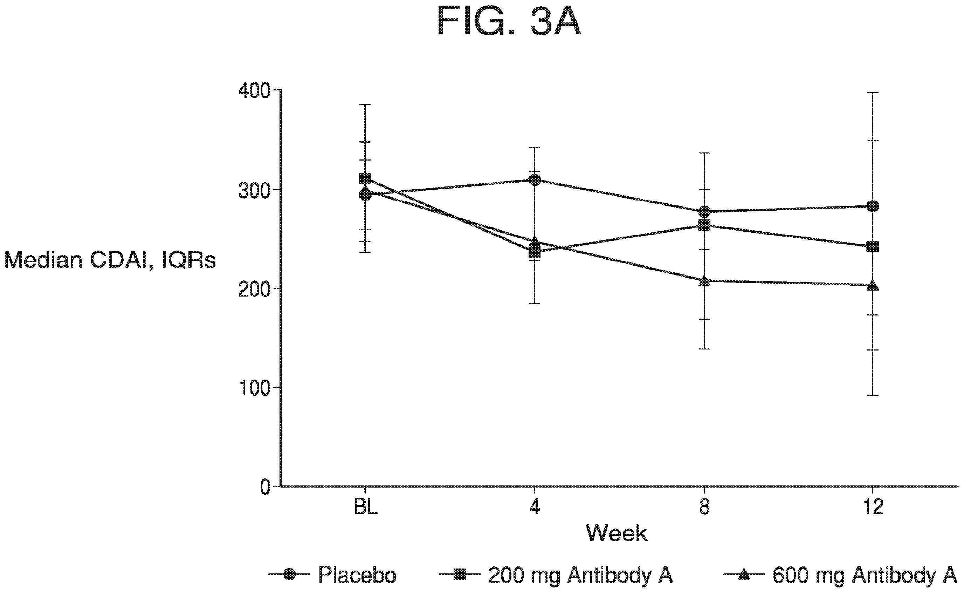

Figure 3A-D: Median CDAI scores over time (A), median CRP over time (B), median percent change in FCP from baseline (C), median percent change in IL-22 from baseline (D). *p <0.001, Bonferroni adjusted p-value for 600 mg Antibody A versus placebo. **p <0.05, Bonferroni adjusted p-value for 600 mg Antibody A versus placebo. CDAI (Crohn's Disease Activity Index); CRP (C-reactive protein); FCP (fecal calprotectin).

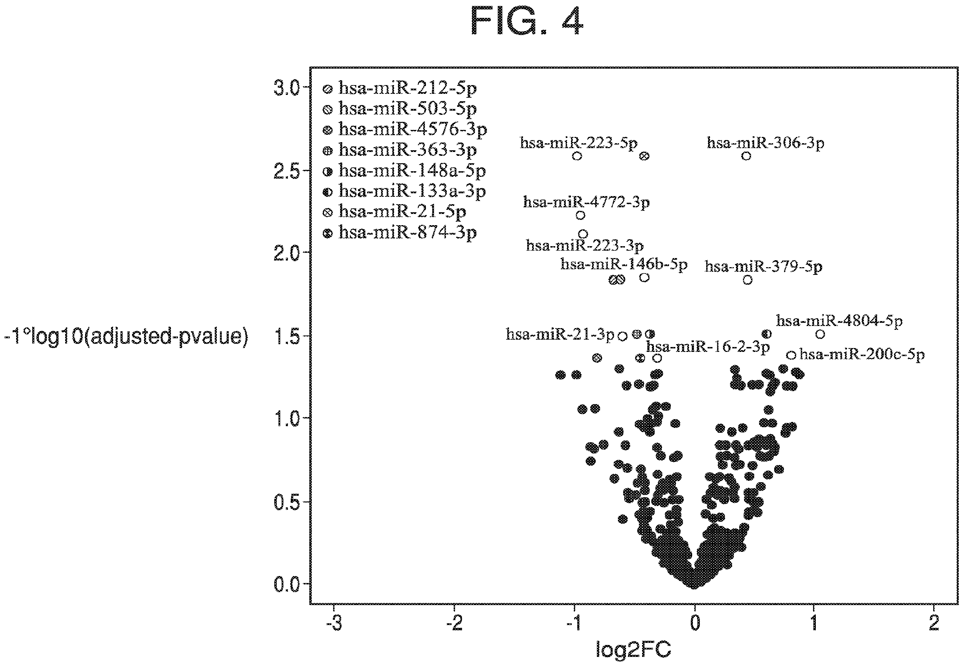

Figure 4: Differentially expressed miRNAs in colon biopsies 12 weeks post-treatment with 600 mg Antibody A treatment (Log2 fold from baseline) versus placebo.

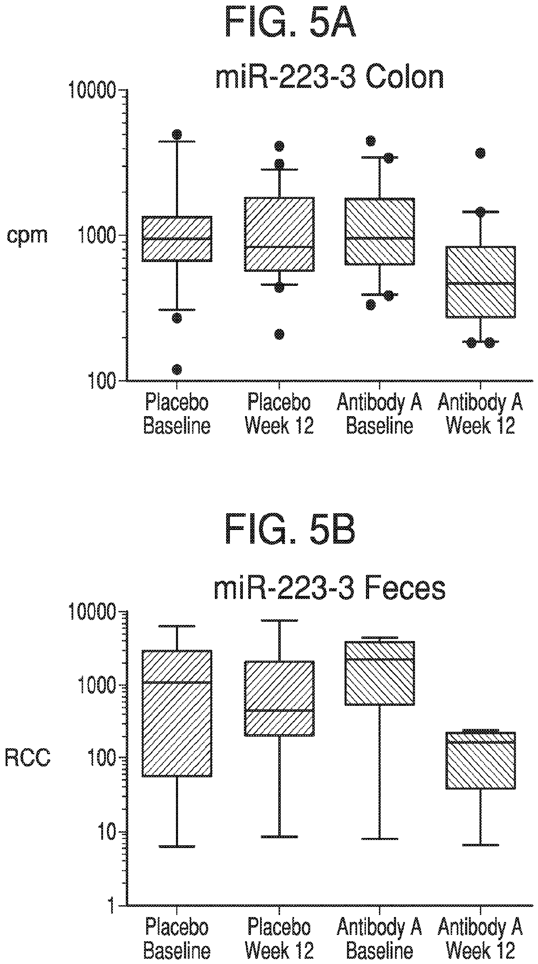

Figure 5A-C: Levels of miR-223-3p in colon (A) and feces (B) in placebo- and Antibody A-treated patients at baseline and week 12. Whisker represent the 10-90 percentiles of expression. Correlation of log fold change in fecal calprotectin levels with log fold change in miR-223-3p expression in colon biopsy tissue in the 600 mg Antibody A dose group (C). *p<0.05; ns, not significant.

### Detailed Description

The p19 subunit of IL-23 (also referred to herein as "IL-23A", "IL-23p19" and "p19 subunit") is a 189 amino acid polypeptide containing a 21 aa leader sequence (Oppmann et al. Immunity 13:715 (2000), SEQ ID NO: 22). The biological activity of the molecule is only detected when it is partnered with the IL-12p40 subunit to form IL-23. IL-23 is predominantly expressed by activated dendritic cells (DCs) and phagocytic cells. The receptor for IL-23 was found to be composed of the IL-12Rβ1 subunit of IL-12 receptor partnered with a unique subunit called IL-23R (Parham et al. J. Immunol. 168:5699 (2002)). Expression of the receptor is detected primarily on memory T cells and NK cells. Thus, expression of this cytokine:receptor pair appears to be restricted to specific populations of immune cells. While it was first thought that IL-12 and IL-23 would share many functions, the data has shown the picture to be different. Whereas IL-12 has a predominant role in the production of Th1 cells, IL-23 was found to be critically involved in the production and maintenance of a recently recognized Th cell subset termed Th17 (Kikly et al. Curr. Opin. Immunol. 18:670 (2006), Kastelein et al. Ann. Rev. Immunol. 25:221 (2007)). These cells produce IL-17A, IL-17F, IL-22 and other proinflammatory cytokines such as IL-6 and TNF-α. As described below, animal model studies on the role of these Th17 cells show their importance as a driving force in chronic inflammation and autoimmunity.
SEQ ID NO: 22:

mlgsravmll lllpwtaqgr avpggsspaw tqcqqlsqkl ctlawsahpl vghmdlreeg
deettndvph iqcgdgcdpq glrdnsqfcl qrihqglify ekllgsdift gepsllpdsp
vgqlhasllg lsqllqpegh hwetqqipsl spsqpwqrll lrfkilrslq afvavaarvf
ahgaatlsp

In one aspect, the present invention provides methods for the treament of IL-23A related diseases. In one aspect, the present invention provides methods for treating a disease, for example an inflammatory disease, in particular methods comprising administering an anti-IL-23A antibody to a patient in certain amounts and/or at certain intervals. In one aspect, a method of the present invention is for the treament of Crohn's Disease.

In one aspect, the present invention provides an anti-IL-23A antibody for use in the treament of a disease, for example an inflammatory disease, for example Crohn's Disease, by administration in certain amounts and/or at certain intervals as described herein.

In one aspect, the present invention provides for the use of an anti-IL-23A antibody for the preparation of a medicament for the treatment of a disease, for example an inflammatory disease, for example Crohn's Disease, by administration in certain amounts and/or at certain intervals as described herein.

In one embodiment, in the context of the present invention, the treatment of a patient comprises an induction phase and a maintenance phase. In the induction phase, one or more doses of an anti-IL-23 antibody, for example referred to herein as induction doses, are administered to the patient, for example by intravenous infusion. In the maintenance phase, a first dose of the anti-IL-23 antibody, for example referred to herein as maintenance dose, is administered to the patient followed by at least one additional dose of the anti-IL-23 antibody, for example referred to herein as maintenance dose. The maintenance doses are for example administered by subcutaneous injection. Examples of induction phases and maintenance phases are described herein.

In one aspect, a certain therapeutic result is achieved by the patient during or at the end of the induction phase, for example clinical remission. In one aspect, a patient achieves a CDAI score of less than 150 during or at the end of the induction phase. In one aspect, a patient achieves a PRO-2 score equal to or less than 75 during or at the end of the induction phase. In one aspect, a patient achieves a CDAI score of less than 150 and a PRO-2 score equal to or less than 75 during or at the end of the induction phase.

Accordingly, in one embodiment, in a method of the present invention, a patient is evaluated for clinical remission during or at the end of the induction phase.

In one aspect, if a patient is not responsive in the induction phase, the induction phase is repeated before the maintenance phase is initiated (also referred herein as re-induction phase). In one aspect, in a method of the present invention, a patient is reevaluated for clinical remission during or at the end of the re-induction phase, for example as during or after the induction phase.

In one aspect, a certain therapeutic result is maintained by the patient during the maintenance phase, for example clinical remission. In one aspect, a patient maintains a CDAI score of less than 150 during the maintenance phase. In one aspect, a patient maintians a PRO-2 score equal to or less than 75 during the maintenance phase. In one aspect, a patient maintains a CDAI score of less than 150 and a PRO-2 score equal to or less than 75 during the maintenance phase.

Accordingly, in one embodiment, in a method of the present invention, a patient is evaluated for clinical remission during the maintenance phase.

In one embodiment, the present invention provides a method for treating an inflammatory disease, in one aspect for treating Crohn's Disease, comprising (a) administering to a patient a dose of an anti-IL-23A antibody at week 0, at week 4 and at week 8 by intravenous infusion, wherein the doses of the anti-IL-23A antibody comprise 200 mg or 600 mg of the antibody. In one embodiment, the method further comprises (b) administering to the patient three doses of the anti-IL-23A antibody, for example at week 14, at week 18 and at week 22 by intravenous infusion, wherein the doses of the anti-IL-23A antibody comprise 600 mg of the antibody. In one embodiment, the method further comprises (c) administering to the patient one or more doses of the anti-IL-23A antibody at 8 weeks intervals by subcutaneous injection, for example four doses of the anti-IL-23A antibody at 8 weeks intervals by subcutaneous injection, for example at week 26, at week 34, at week 42 and at week 50, wherein the one or more doses of the anti-IL-23A antibody comprise 180 mg of the antibody.

In one embodiment, at week 12, a patient is evaluated for deep remission, for example defined as reaching clinical remission (CDAI score <150) and endoscopic remission (CDEIS ≤ 4). In one, aspect, for a patient with initial isolated ileitis endoscopic remission is defined by CDEIS ≤ 2.

In one embodiment, the present invention provides a method for treating an inflammatory disease, in one aspect for treating Crohn's Disease, comprising (a) administering to a patient a dose of an anti-IL-23A antibody at week 0, at week 4 and at week 8 by intravenous infusion, wherein the doses of the anti-IL-23A antibody comprise 200 mg or 600 mg of the antibody. In one embodiment, the method further comprises (b) administering to the patient one or more doses of the anti-IL-23A antibody at 8 weeks intervals by subcutaneous injection, for example four doses of the anti-IL-23A antibody at 8 weeks intervals by subcutaneous injection, for example at week 26, at week 34, at week 42 and at week 50, wherein the one or more doses of the anti-IL-23A antibody comprise 180 mg of the antibody.

In one embodiment, at week 12, a patient is evaluated for deep remission, for example defined as reaching clinical remission (CDAI score <150) and endoscopic remission (CDEIS ≤ 4). In one, aspect, for a patient with initial isolated ileitis endoscopic remission is defined by CDEIS ≤ 2.

In one embodiment, the present invention provides a method for treating an inflammatory disease, in one aspect for treating Crohn's Disease, comprising administering to a patient 180 mg of the anti-IL-23A antibody at 8 weeks intervals by subcutaneous injection.

In one embodiment, the administration of an anti-23A antibody according to the present invention is further described in the Examples hereinbelow or in Figure 1.

In one embodiment, the present invention provides a method for treating an inflammatory disease, in one aspect for treating Crohn's Disease, comprising administering to a patient an anti-IL-23A antibody, said method comprising administering at least one induction dose of the anti-IL-23A antibody to the patient, wherein said induction dose comprises 200 to 1,200 mg of the anti-IL-23A antibody, for example 450 to 1,200 mg of the anti-IL-23A antibody. In one aspect, the induction dose comprises 200 mg, 450 mg, 600 mg, 900 mg or 1,200 mg of the anti-IL-23A antibody. In one embodiment, the induction dose comprises 750 mg of said anti-IL-23A antibody. In one aspect, 1, 2 or 3 induction doses are administered to the patient. In one aspect, 2 or 3 inductions doses are administered, for example at 4 weeks intervals. In one aspect, the induction dose(s) is administered by intravenous infusion.

In one embodiment, the induction doses comprise 200 mg of the anti-IL-23A antibody and three inductions does are administered to the patient at 4 weeks intervals.

In one embodiment, the induction doses comprise 450 mg of the anti-IL-23A antibody and three inductions does are administered to the patient at 4 weeks intervals.

In one embodiment, the induction doses comprise 600 mg of the anti-IL-23A antibody and three inductions does are administered to the patient at 4 weeks intervals.

In one embodiment, the induction doses comprise 900 mg of the anti-IL-23A antibody and three inductions does are administered to the patient at 4 weeks intervals.

In one embodiment, the induction doses comprise 1,200 mg of the anti-IL-23A antibody and three inductions does are administered to the patient at 4 weeks intervals.

In one embodiment, the induction doses comprise 750 mg of the anti-IL-23A antibody and three inductions does are administered to the patient at 4 weeks intervals.

In one aspect, if a patient is not responsive in the induction phase, the induction phase is repeated before the maintenance phase is initiated. For example, if the first induction phase included three induction doses, three additional induction doses are administered to the patient (re-induction phase), leading to a total of six induction doses. The additional induction dose(s) comprise(s) for example an amount of the anti-IL-23 antibody described herein. The additional induction doses are for example administered at an interval described herein.

In one ambodiment, the method further comprises administering a first maintenance dose of the anti-IL-23A antibody to the patient after the last induction dose is administered; and administering at least one additional maintenance dose to the patient 4 to 12 weeks after said first maintenance dose is administered. In one aspect, the first maintenance dose is administered 2 to 8 weeks, for example 4 to 6 weeks, for example 2 weeks, 4 weeks, 6 weeks or 8 weeks, after the last induction dose is administered. In one aspect, the at least one additional maintenance dose is administered to the patient 4, 8 or 12 weeks after the first maintenance dose is administered.

In one embodiment, the first maintenance dose comprises 150 to 300 mg of the anti-IL-23A antibody. In one aspect, the first maintenance dose comprises 150 mg, 225 mg or 300 mg of the anti-IL-23A antibody. In one aspect, the first maintenance dose comprises 180 mg or 270 mg of the anti-IL-23A antibody.

In one aspect, the at least one additional maintenance dose comprises 150 to 300 mg of the anti-IL-23A antibody. In one aspect, the at least one additional maintenance dose comprises 150 mg, 225 mg or 300 mg of the anti-IL-23A antibody. In one aspect, the at least one additional maintenance dose comprises 180 mg or 270 mg of the anti-IL-23A antibody.

In one aspect, the first maintenance dose and the at least one additional maintenance dose comprise 150 to 300 mg of the anti-IL-23A antibody. In one aspect, the first maintenance dose and the at least one additional maintenance dose comprise 150 mg, 225 mg or 300 mg of said anti-IL-23A antibody. In one aspect, the first maintenance dose and the at least one additional maintenance dose comprise 180 mg or 270 mg of said anti-IL-23A antibody.

In one aspect, the maintenance dose is administered by subcutaneous injection.

In one embodiment, the maintenance doses comprise 150 mg of the anti-IL-23A antibody and are administered to the patient at 4 weeks intervals.

In one embodiment, the maintenance doses comprise 150 mg of the anti-IL-23A antibody and are administered to the patient at 8 weeks intervals.

In one embodiment, the maintenance doses comprise 225 mg of the anti-IL-23A antibody and are administered to the patient at 8 weeks intervals.

In one embodiment, the maintenance doses comprise 225 mg of the anti-IL-23A antibody and are administered to the patient at 12 weeks intervals.

In one embodiment, the maintenance doses comprise 300 mg of the anti-IL-23A antibody and are administered to the patient at 8 weeks intervals.

In one embodiment, the maintenance doses comprise 300 mg of the anti-IL-23A antibody and are administered to the patient at 12 weeks intervals.

In one embodiment, the present invention provides a method for treating an inflammatory disease, in one aspect for treating Crohn's Disease, comprising administering to a patient 150 to 1,200 mg of an anti-IL-23A antibody. In one aspect, the method comprises administering to a patient 200 to 1,200 mg of an anti-IL-23A antibody, for example 450 to 1,200 mg of an anti-IL-23A antibody. In one aspect, the method comprises administering to a patient 200 mg, 450 mg, 600 mg, 900 mg or 1,200 mg of an anti-IL-23A antibody. In one aspect, the method comprises administering to a patient 750 mg of an anti-IL-23A antibody. In one aspect, the method comprises administering to a patient 150 to 300 mg of an anti-IL-23A antibody. In one aspect, the method comprises administering to a patient 150 mg, 225 mg or 300 mg of an anti-IL-23A antibody. In one aspect, the method comprises administering to a patient 180 mg or 270 mg of an anti-IL-23A antibody.

In one embodiment, the present invention further provides a method for inducing clinical remission of Crohn's Disease in a patient, said method comprising administering to the patient at least one induction dose of an anti-IL-23 antibody as described above or herein. In one embodiment, the method further comprising maintaining clinical remission of Crohn's disease, said method further comprising administering a first maintenance dose of said anti-IL-23A antibody to the patient after the last induction dose is administered and administering at least one additional maintenance dose to the patient as described above or herein.

In one embodiment, the present invention further provides a method for inducing clinical response to Crohn's Disease in a patient, said method comprising administering to the patient at least one induction dose of an anti-IL-23 antibody as described above or herein. In one embodiment, the method further comprising maintaining clinical response to Crohn's disease, said method further comprising administering a first maintenance dose of said anti-IL-23A antibody to the patient after the last induction dose is administered and administering at least one additional maintenance dose to the patient as described above or herein.

Representative examples of doses and dose regimens according to the present invention are disclosed in Table A.

**Table A: doses and dose regimens**

| Induction dose (mg) | Frequency of induction doses | Maintenance dose (mg) | Frequency of maintenance doses |
|---|---|---|---|
| 450 | Every 4 weeks | 150 | Every 4 weeks |
| 450 | Every 4 weeks | 150 | Every 8 weeks |
| 450 | Every 4 weeks | 180 | Every 4 weeks |
| 450 | Every 4 weeks | 180 | Every 8 weeks |
| 450 | Every 4 weeks | 225 | Every 8 weeks |
| 450 | Every 4 weeks | 225 | Every 12 weeks |
| 450 | Every 4 weeks | 270 | Every 8 weeks |
| 450 | Every 4 weeks | 270 | Every 12 weeks |
| 450 | Every 4 weeks | 300 | Every 8 weeks |
| 450 | Every 4 weeks | 300 | Every 12 weeks |
| 600 | Every 4 weeks | 150 | Every 4 weeks |
| 600 | Every 4 weeks | 150 | Every 8 weeks |
| 600 | Every 4 weeks | 180 | Every 4 weeks |
| 600 | Every 4 weeks | 180 | Every 8 weeks |
| 600 | Every 4 weeks | 225 | Every 8 weeks |
| 600 | Every 4 weeks | 225 | Every 12 weeks |
| 600 | Every 4 weeks | 270 | Every 8 weeks |
| 600 | Every 4 weeks | 270 | Every 12 weeks |
| 600 | Every 4 weeks | 300 | Every 8 weeks |
| 600 | Every 4 weeks | 300 | Every 12 weeks |
| 900 | Every 4 weeks | 150 | Every 4 weeks |
| 900 | Every 4 weeks | 150 | Every 8 weeks |
| 900 | Every 4 weeks | 180 | Every 4 weeks |
| 900 | Every 4 weeks | 180 | Every 8 weeks |
| 900 | Every 4 weeks | 225 | Every 8 weeks |
| 900 | Every 4 weeks | 225 | Every 12 weeks |
| 900 | Every 4 weeks | 270 | Every 8 weeks |
| 900 | Every 4 weeks | 270 | Every 12 weeks |
| 900 | Every 4 weeks | 300 | Every 8 weeks |
| 900 | Every 4 weeks | 300 | Every 12 weeks |
| 1,200 | Every 4 weeks | 150 | Every 4 weeks |
| 1,200 | Every 4 weeks | 150 | Every 8 weeks |
| 1,200 | Every 4 weeks | 180 | Every 4 weeks |
| 1,200 | Every 4 weeks | 180 | Every 8 weeks |
| 1,200 | Every 4 weeks | 225 | Every 8 weeks |
| 1,200 | Every 4 weeks | 225 | Every 12 weeks |
| 1,200 | Every 4 weeks | 270 | Every 8 weeks |
| 1,200 | Every 4 weeks | 270 | Every 12 weeks |
| 1,200 | Every 4 weeks | 300 | Every 8 weeks |
| 1,200 | Every 4 weeks | 300 | Every 12 weeks |
| 750 | Every 4 weeks | 150 | Every 4 weeks |
| 750 | Every 4 weeks | 150 | Every 8 weeks |
| 750 | Every 4 weeks | 180 | Every 4 weeks |
| 750 | Every 4 weeks | 180 | Every 8 weeks |
| 750 | Every 4 weeks | 225 | Every 8 weeks |
| 750 | Every 4 weeks | 225 | Every 12 weeks |
| 750 | Every 4 weeks | 270 | Every 8 weeks |
| 750 | Every 4 weeks | 270 | Every 12 weeks |
| 750 | Every 4 weeks | 300 | Every 8 weeks |
| 750 | Every 4 weeks | 300 | Every 12 weeks |

In one aspect, 1, 2 or 3 induction dose(s) is/are administered to the patient in a dose regimen described in Table A.

Additional representative examples of doses and dose regimens according to the present invention are described below. In these examples, the first maintenance dose is administered is administered to the patient 4 weeks after the last induction dose. It is also contemplated in the present invention to administer the first maintenance dose 2 weeks, 6 weeks or 8 weeks after the last induction dose.

For example, in the context of the present invention, inductions doses are administered to the patient at week 0, week 4 and week 8, followed by a first maintenance dose at week 12, a second maintenance dose at week 16, a third maintenance dose at week 20 and so on at dosing intervals of 4 weeks between the maintenance doses. In one aspect, the inductions doses comprise 200 mg, 450 mg, 600 mg, 900 mg or 1,200 mg of the anti-IL-23A antibody. In one aspect, the maintenance doses comprise 150 mg of the anti-IL-23A antibody.

For example, in the context of the present invention, inductions doses are administered to the patient at week 0, week 4 and week 8, followed by a first maintenance dose at week 12, a second maintenance dose at week 20, a third maintenance dose at week 28 and so on at dosing intervals of 8 weeks between the maintenance doses. In one aspect, the inductions doses comprise 200 mg, 450 mg, 600 mg, 900 mg or 1,200 mg of the anti-IL-23A antibody. In one aspect, the maintenance doses comprise 150 mg, 225 mg or 300 mg of the anti-IL-23A antibody.

For example, in the context of the present invention, inductions doses are administered to the patient at week 0, week 4 and week 8, followed by a first maintenance dose at week 12, a second maintenance dose at week 24, a third maintenance dose at week 36 and so on at dosing intervals of 12 weeks between the maintenance doses. In one aspect, the inductions doses comprise 200 mg, 450 mg, 600 mg, 900 mg or 1,200 mg of the anti-IL-23A antibody. In one aspect, the maintenance doses comprise 225 mg or 300 mg of the anti-IL-23A antibody.

In one embodiment, in a method of the present invention, a patient is evaluated for deep remission, for example defined as reaching clinical remission (CDAI score <150) and endoscopic remission (CDEIS ≤ 4). In one aspect, for a patient with initial isolated ileitis endoscopic remission is defined by CDEIS ≤ 2. In one aspect, the PRO response of the patient is assessed, for example defined by either a PRO-2 score of <8 or a reduction from baseline of at least 8 points (PRO-2: Patient reported outcome-2).

In one embodiment, in a method of the present invention, a patient is evaluated for clinical remission, clinical response, endoscopic remission, endoscopic response or mucosal healing, for example as defined herein,

In one embodiment, an anti-IL-23A antibody in any one of the methods above is disclosed herein. In one embodiment, an anti-IL-23A antibody in any one of the methods above is Antibody A. In one embodiment, an anti-IL-23A antibody in any one of the methods above is Antibody B. In one embodiment, an anti-IL-23A antibody in any one of the methods above is Antibody C. In one embodiment, an anti-IL-23A antibody in any one of the methods above is Antibody D.

In one aspect, in any one of the methods above, a pharmaceutical composition comprising an anti-IL-23A antibody is administered to the patient. In one aspect, formulation 2 disclosed in Example 2 comprising an anti-IL-23A antibody, for example Antibody A, Antibody B, Antibody C or Antibody D is administered to the patient. In one aspect, formulation 3 disclosed in Example 2 comprising an anti-IL-23A antibody, for example Antibody A, Antibody B, Antibody C or Antibody D is administered to the patient. In one aspect, formulation 1 disclosed in Example 2 comprising an anti-IL-23A antibody, for example Antibody A, Antibody B, Antibody C or Antibody D is administered to the patient.

In one aspect, the anti-IL-23A antibody is a humanized antibody. In one aspect, the anti-IL-23A antibody is a monoclonal antibody. In one aspect, the anti-IL-23A antibody is a full length antibody. In one aspect, the anti-IL-23A antibody is a humanized monoclonal antibody, for example a full length humanized monoclonal antibody.

An antibody described herein recognizes specific "IL-23A antigen epitope" or " IL-23A epitope". As used herein these terms refer to a molecule (e.g., a peptide) or a fragment of a molecule capable of immunoreactivity with an anti-IL-23A antibody and, for example, include an IL-23A antigenic determinant recognized by any of the antibodies having a light chain/heavy chain sequence combination of SEQ ID NO:11/14, 11/15, 10/14 or 10/15.

The generalized structure of antibodies or immunoglobulin is well known to those of skill in the art. These molecules are heterotetrameric glycoproteins, typically of about 150,000 daltons, composed of two identical light (L) chains and two identical heavy (H) chains and are typically referred to as full length antibodies. Each light chain is covalently linked to a heavy chain by one disulfide bond to form a heterodimer, and the heterotrameric molecule is formed through a covalent disulfide linkage between the two identical heavy chains of the heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (V_{H}), followed by three or four constant domains (C_{H1}, C_{H2}, C_{H3}, and C_{H4}), as well as a hinge region between C_{H1} and C_{H2}. Each light chain has two domains, an amino-terminal variable domain (V_{L}) and a carboxy-terminal constant domain (C_{L}). The V_{L} domain associates non-covalently with the V_{H} domain, whereas the C_{L} domain is commonly covalently linked to the C_{H1} domain via a disulfide bond. Particular amino acid residues are believed to form an interface between the light and heavy chain variable domains (Chothia et al., 1985, J. Mol. Biol. 186:651-663). Variable domains are also referred herein as variable regions.

Certain domains within the variable domains differ extensively between different antibodies i.e., are "hypervariable." These hypervariable domains contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat et al., 1991, In: Sequences of Proteins of Immunological Interest, 5th Ed. Public Health Service, National Institutes of Health, Bethesda, Md., whereas HVLs (also referred herein as CDRs) are structurally defined according to the three-dimensional structure of the variable domain, as described by Chothia and Lesk, 1987, J. Mol. Biol. 196: 901-917. These two methods result in slightly different identifications of a CDR. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain. The exact residue numbers that encompass a particular CDR will vary depending on the sequence and size of the CDR. Those skilled in the art can routinely determine which residues comprise a particular CDR given the variable region amino acid sequence of the antibody. The CDR1, CDR2, CDR3 of the heavy and light chains therefore define the unique and functional properties specific for a given antibody.

The three CDRs within each of the heavy and light chains are separated by framework regions (FR), which contain sequences that tend to be less variable. From the amino terminus to the carboxy terminus of the heavy and light chain variable domains, the FRs and CDRs are arranged in the order: FR1, CDR1, FR2, CDR2, FR3, CDR3, and FR4. The largely β-sheet configuration of the FRs brings the CDRs within each of the chains into close proximity to each other as well as to the CDRs from the other chain. The resulting conformation contributes to the antigen binding site (see Kabat et al., 1991, NIH Publ. No. 91-3242, Vol. I, pages 647-669), although not all CDR residues are necessarily directly involved in antigen binding.

FR residues and Ig constant domains are not directly involved in antigen binding, but contribute to antigen binding and/or mediate antibody effector function. Some FR residues are thought to have a significant effect on antigen binding in at least three ways: by noncovalently binding directly to an epitope, by interacting with one or more CDR residues, and by affecting the interface between the heavy and light chains. The constant domains are not directly involved in antigen binding but mediate various Ig effector functions, such as participation of the antibody in antibody dependent cellular cytotoxicity (ADCC), complement dependent cytotoxicity (CDC) and antibody dependent cellular phagocytosis (ADCP).

The light chains of vertebrate immunoglobulins are assigned to one of two clearly distinct classes, kappa (κ) and lambda (λ), based on the amino acid sequence of the constant domain. By comparison, the heavy chains of mammalian immunoglobulins are assigned to one of five major classes, according to the sequence of the constant domains: IgA, IgD, IgE, IgG, and IgM. IgG and IgA are further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of the classes of native immunoglobulins are well known.

The terms, "antibody", "anti-IL-23A antibody", "anti-IL-23p19 antibody", "humanized anti-IL-23A antibody", "humanized anti-IL-23p19 antibody", "humanized anti-IL-23A epitope antibody", humanized anti-IL-2319 epitope antibody", "variant humanized anti-IL-23A epitope antibody" and "variant humanized anti-IL-23p19 epitope antibody" specifically encompass monoclonal antibodies (including full length monoclonal antibodies), polyclonal antibodies, and antibody fragments such as variable domains and other portions of antibodies that exhibit a desired biological activity, e.g., IL-23A binding. The term "monoclonal antibody" (mAb) refers to an antibody that is highly specific, being directed against a single antigenic determinant, an "epitope". Therefore, the modifier "monoclonal" is indicative of antibodies directed to the identical epitope and is not to be construed as requiring production of the antibody by any particular method. It should be understood that monoclonal antibodies can be made by any technique or methodology known in the art; including e.g., the hybridoma method ( Kohler et al., 1975, Nature 256:495), or recombinant DNA methods known in the art (see, e.g., U.S. Pat. No. 4,816,567), or methods of isolation of monoclonal recombinantly produced using phage antibody libraries, using techniques described in Clackson et al., 1991, Nature 352: 624-628, and Marks et al., 1991, J. Mol. Biol. 222: 581-597.

The term "monomer" refers to a homogenous form of an antibody. For example, for a full-length antibody, monomer means a monomeric antibody having two identical heavy chains and two identical light chains.

Chimeric antibodies consist of the heavy and light chain variable regions of an antibody from one species (e.g., a non-human mammal such as a mouse) and the heavy and light chain constant regions of another species (e.g., human) antibody and can be obtained by linking the DNA sequences encoding the variable regions of the antibody from the first species (e.g., mouse) to the DNA sequences for the constant regions of the antibody from the second (e.g. human) species and transforming a host with an expression vector containing the linked sequences to allow it to produce a chimeric antibody. Alternatively, the chimeric antibody also could be one in which one or more regions or domains of the heavy and/or light chain is identical with, homologous to, or a variant of the corresponding sequence in a monoclonal antibody from another immunoglobulin class or isotype, or from a consensus or germline sequence. Chimeric antibodies can include fragments of such antibodies, provided that the antibody fragment exhibits the desired biological activity of its parent antibody, for example binding to the same epitope (see, e.g., U.S. Pat. No. 4,816,567; and Morrison et al., 1984, Proc. Natl. Acad. Sci. USA 81: 6851-6855).

The terms, "antibody fragment", "anti-IL-23A antibody fragment", "anti-IL-23A epitope antibody fragment", "humanized anti-IL-23A antibody fragment", "humanized anti-IL-23A epitope antibody fragment", "variant humanized anti-IL-23A epitope antibody fragment" refer to a portion of a full length anti-IL-23A antibody, in which a variable region or a functional capability is retained, for example, specific IL-23A epitope binding. Examples of antibody fragments include, but are not limited to, a Fab, Fab', F(ab')₂, Fd, Fv, scFv and scFv-Fc fragment.

Full length antibodies can be treated with enzymes such as papain or pepsin to generate useful antibody fragments. Papain digestion is used to produces two identical antigen-binding antibody fragments called "Fab" fragments, each with a single antigen-binding site, and a residual "Fc" fragment. The Fab fragment also contains the constant domain of the light chain and the C_{H1} domain of the heavy chain. Pepsin treatment yields a F(ab')₂ fragment that has two antigen-binding sites and is still capable of crosslinking antigen.

Fab' fragments differ from Fab fragments by the presence of additional residues including one or more cysteines from the antibody hinge region at the C-terminus of the C_{H1} domain. F(ab')₂ antibody fragments are pairs of Fab' fragments linked by cysteine residues in the hinge region. Other chemical couplings of antibody fragments are also known.

"Fv" fragment contains a complete antigen-recognition and binding site consisting of a dimer of one heavy and one light chain variable domain in tight, non-covalent association. In this configuration, the three CDRs of each variable domain interact to define an antigen-biding site on the surface of the V_{H}-V_{L} dimer. Collectively, the six CDRs confer antigen-binding specificity to the antibody.

A "single-chain Fv" or "scFv" antibody fragment is a single chain Fv variant comprising the V_{H} and V_{L} domains of an antibody where the domains are present in a single polypeptide chain. The single chain Fv is capable of recognizing and binding antigen. The scFv polypeptide may optionally also contain a polypeptide linker positioned between the V_{H} and V_{L} domains in order to facilitate formation of a desired three-dimensional structure for antigen binding by the scFv (see, e.g., Pluckthun, 1994, In The Pharmacology of monoclonal Antibodies, Vol. 113, Rosenburg and Moore eds., Springer-Verlag, New York, pp. 269-315).

A "humanized antibody" or a "humanized antibody fragment" is a specific type of chimeric antibody which includes an immunoglobulin amino acid sequence variant, or fragment thereof, which is capable of binding to a predetermined antigen and which, comprises one or more FRs having substantially the amino acid sequence of a human immunoglobulin and one or more CDRs having substantially the amino acid sequence of a non-human immunoglobulin. This non-human amino acid sequence often referred to as an "import" sequence is typically taken from an "import" antibody domain, particularly a variable domain. In general, a humanized antibody includes at least the CDRs or HVLs of a non-human antibody, inserted between the FRs of a human heavy or light chain variable domain. The present invention describes specific humanized anti-IL-23A antibodies which contain CDRs derived from the mouse monoclonal antibodies or humanized CDRs shown in Tables 1 and 2 inserted between the FRs of human germline sequence heavy and light chain variable domains. It will be understood that certain mouse FR residues may be important to the function of the humanized antibodies and therefore certain of the human germline sequence heavy and light chain variable domains residues are modified to be the same as those of the corresponding mouse sequence.

In another aspect, a humanized anti-IL-23A antibody comprises substantially all of at least one, and typically two, variable domains (such as contained, for example, in Fab, Fab', F(ab')2, Fabc, and Fv fragments) in which all, or substantially all, of the CDRs correspond to those of a non-human immunoglobulin, and specifically herein, all of the CDRs are mouse or humanized sequences as detailed in Tables 1 and 2 herein below and all, or substantially all, of the FRs are those of a human immunoglobulin consensus or germline sequence. In another aspect, a humanized anti- IL-23A antibody also includes at least a portion of an immunoglobulin Fc region, typically that of a human immunoglobulin. Ordinarily, the antibody will contain both the light chain as well as at least the variable domain of a heavy chain. The antibody also may include one or more of the C_{H1}, hinge, C_{H2}, C_{H3}, and/or C_{H4} regions of the heavy chain, as appropriate.

A humanized anti-IL-23A antibody can be selected from any class of immunoglobulins, including IgM, IgG, IgD, IgA and IgE, and any isotype, including IgG₁, IgG₂, IgG₃, IgG₄, IgA₁ and IgA₂. For example, the constant domain can be a complement fixing constant domain where it is desired that the humanized antibody exhibit cytotoxic activity, and the isotype is typically IgG₁. Where such cytotoxic activity is not desirable, the constant domain may be of another isotype, e.g., IgG₂. An alternative humanized anti-IL-23A antibody can comprise sequences from more than one immunoglobulin class or isotype, and selecting particular constant domains to optimize desired effector functions is within the ordinary skill in the art. In specific embodiments, the present invention provides antibodies that are IgG1 antibodies and more particularly, are IgG1 antibodies in which there is a knock-out of effector functions.

The FRs and CDRs, or HVLs, of a humanized anti-IL-23A antibody need not correspond precisely to the parental sequences. For example, one or more residues in the import CDR, or HVL, or the consensus or germline FR sequence may be altered (e.g., mutagenized) by substitution, insertion or deletion such that the resulting amino acid residue is no longer identical to the original residue in the corresponding position in either parental sequence but the antibody nevertheless retains the function of binding to IL-23A. Such alteration typically will not be extensive and will be conservative alterations. Usually, at least 75% of the humanized antibody residues will correspond to those of the parental consensus or germline FR and import CDR sequences, more often at least 90%, and most frequently greater than 95%, or greater than 98% or greater than 99%.

Immunoglobulin residues that affect the interface between heavy and light chain variable regions ("the V_{L}-V_{H} interface") are those that affect the proximity or orientation of the two chains with respect to one another. Certain residues that may be involved in interchain interactions include V_{L} residues 34, 36, 38, 44, 46, 87, 89, 91, 96, and 98 and V_{H} residues 35, 37, 39, 45, 47, 91, 93, 95, 100, and 103 (utilizing the numbering system set forth in Kabat et al., Sequences of Proteins of Immunological Interest (National Institutes of Health, Bethesda, Md., 1987)). U.S. Pat. No. 6,407,213 also discusses that residues such as V_{L} residues 43 and 85, and V_{H} residues 43 and 60 also may be involved in this interaction. While these residues are indicated for human IgG only, they are applicable across species. Important antibody residues that are reasonably expected to be involved in interchain interactions are selected for substitution into the consensus sequence.

The terms "consensus sequence" and "consensus antibody" refer to an amino acid sequence which comprises the most frequently occurring amino acid residue at each location in all immunoglobulins of any particular class, isotype, or subunit structure, e.g., a human immunoglobulin variable domain. The consensus sequence may be based on immunoglobulins of a particular species or of many species. A "consensus" sequence, structure, or antibody is understood to encompass a consensus human sequence as described in certain embodiments, and to refer to an amino acid sequence which comprises the most frequently occurring amino acid residues at each location in all human immunoglobulins of any particular class, isotype, or subunit structure. Thus, the consensus sequence contains an amino acid sequence having at each position an amino acid that is present in one or more known immunoglobulins, but which may not exactly duplicate the entire amino acid sequence of any single immunoglobulin. The variable region consensus sequence is not obtained from any naturally produced antibody or immunoglobulin. Kabat et al., 1991, Sequences of Proteins of Immunological Interest, 5th Ed. Public Health Service, National Institutes of Health, Bethesda, Md., and variants thereof. The FRs of heavy and light chain consensus sequences, and variants thereof, provide useful sequences for the preparation of humanized anti-IL-23p19 antibodies. See, for example, U.S. Pat. Nos. 6,037,454 and 6,054,297.

Human germline sequences are found naturally in the human population. A combination of those germline genes generates antibody diversity. Germline antibody sequences for the light chain of the antibody come from conserved human germline kappa or lambda v-genes and j-genes. Similarly the heavy chain sequences come from germline v-, d- and j-genes (LeFranc, M-P, and LeFranc, G, "The Immunoglobulin Facts Book" Academic Press, 2001).

As used herein, "variant", "anti- IL-23A variant", "humanized anti- IL-23A variant", or "variant humanized anti- IL-23A" each refers to a humanized anti-IL-23A antibody having at least a light chain variable murine CDR from any of the sequences as shown in Table 1 or a heavy chain murine CDR sequence derived from the murine monoclonal antibody as shown in Table 2. Variants include those having one or more amino acid changes in one or both light chain or heavy chain variable domains, provided that the amino acid change does not substantially impair binding of the antibody to IL-23A. Exemplary antibodies produced herein include those designated as Antibody A, Antibody B, Antibody C and Antibody D, and the various light chains and heavy chains of the same are shown in SEQ ID Nos:18 and 21, and SEQ ID Nos:19 and 20, respectively.

An "isolated" antibody is one that has been identified and separated and/or recovered from a component of its natural environment. Contaminant components of the antibody's natural environment are those materials that may interfere with diagnostic or therapeutic uses of the antibody, and can be enzymes, hormones, or other proteinaceous or nonproteinaceous solutes. In one aspect, the antibody will be purified to at least greater than 95% isolation by weight of antibody.

An isolated antibody includes an antibody in situ within recombinant cells in which it is produced, since at least one component of the antibody's natural environment will not be present. Ordinarily however, an isolated antibody will be prepared by at least one purification step in which the recombinant cellular material is removed.

The term "antibody performance" refers to factors that contribute to antibody recognition of antigen or the effectiveness of an antibody in vivo. Changes in the amino acid sequence of an antibody can affect antibody properties such as folding, and can influence physical factors such as initial rate of antibody binding to antigen (kₐ), dissociation constant of the antibody from antigen (k_{d}), affinity constant of the antibody for the antigen (Kd), conformation of the antibody, protein stability, and half life of the antibody.

The term "epitope tagged" when used herein, refers to an anti-IL-23A antibody fused to an "epitope tag". An "epitope tag" is a polypeptide having a sufficient number of amino acids to provide an epitope for antibody production, yet is designed such that it does not interfere with the desired activity of the anti-IL-23A antibody. The epitope tag is usually sufficiently unique such that an antibody raised against the epitope tag does not substantially cross-react with other epitopes. Suitable tag polypeptides generally contain at least 6 amino acid residues and usually contain about 8 to 50 amino acid residues, or about 9 to 30 residues. Examples of epitope tags and the antibody that binds the epitope include the flu HA tag polypeptide and its antibody 12CA5 (Field et al., 1988 Mol. Cell. Biol. 8: 2159-2165; c-myc tag and 8F9, 3C7, 6E10, G4, B7 and 9E10 antibodies thereto (Evan et al., 1985, Mol. Cell. Biol. 5(12):3610-3616; and Herpes simplex virus glycoprotein D (gD) tag and its antibody (Paborsky et al. 1990, Protein Engineering 3(6): 547-553). In certain embodiments, the epitope tag is a "salvage receptor binding epitope". As used herein, the term "salvage receptor binding epitope" refers to an epitope of the Fc region of an IgG molecule (such as IgG₁, IgG₂, IgG₃, or IgG₄) that is responsible for increasing the in vivo serum half-life of the IgG molecule.

For diagnostic as well as therapeutic monitoring purposes, the antibodies of the invention also may be conjugated to a label, either a label alone or a label and an additional second agent (prodrug, chemotherapeutic agent and the like). A label, as distinguished from the other second agents refers to an agent that is a detectable compound or composition and it may be conjugated directly or indirectly to a antibody of the present invention. The label may itself be detectable (e.g., radioisotope labels or fluorescent labels) or, in the case of an enzymatic label, may catalyze chemical alteration of a substrate compound or composition that is detectable. Labeled anti-IL-23A antibody can be prepared and used in various applications including in vitro and in vivo diagnostics.

In various aspects of the present invention one or more domains of the antibodies will be recombinantly expressed. Such recombinant expression may employ one or more control sequences, i.e., polynucleotide sequences necessary for expression of an operably linked coding sequence in a particular host organism. The control sequences suitable for use in prokaryotic cells include, for example, promoter, operator, and ribosome binding site sequences. Eukaryotic control sequences include, but are not limited to, promoters, polyadenylation signals, and enhancers. These control sequences can be utilized for expression and production of anti-IL-23A antibody in prokaryotic and eukaryotic host cells.

A nucleic acid sequence is "operably linked" when it is placed into a functional relationship with another nucleic acid sequence. For example, a nucleic acid presequence or secretory leader is operably linked to a nucleic acid encoding a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers are optionally contiguous. Linking can be accomplished by ligation at convenient restriction sites. If such sites do not exist, synthetic oligonucleotide adaptors or linkers can be used.

As used herein, the expressions "cell", "cell line", and "cell culture" are used interchangeably and all such designations include the progeny thereof. Thus, "transformants" and "transformed cells" include the primary subject cell and cultures derived therefrom without regard for the number of transfers.

The term "mammal" for purposes of treatment refers to any animal classified as a mammal, including humans, domesticated and farm animals, and zoo, sports, or pet animals, such as dogs, horses, cats, cows, and the like. Preferably, the mammal is human.

A "disorder", as used herein, is any condition that would benefit from treatment with an anti-IL-23A antibody described herein. This includes chronic and acute disorders or diseases including those pathological conditions that predispose the mammal to the disorder in question.

As used herein, the term "IL-23-associated disorder" or "IL-23-associated disease" refers to a condition in which IL-23 activity contributes to the disease and typically where IL-23 is abnormally expressed. An IL-23-associated disorder includes diseases and disorders of the immune system, such as autoimmune disorders and inflammatory diseases. Such conditions include psoriasis, inflammatory bowel disease, for example ulcerative colitis or Crohn's disease, and spondyloarthritis, for example ankylosing spondylitis, non-radiographic axial spondyloarthritis, peripheral spondyloarthritis or psoriatic arthritis.

The term "intravenous infusion" refers to introduction of an agent into the vein of an animal or human patient over a period of time greater than approximately 15 minutes, generally between approximately 30 to 90 minutes.

The term "intravenous bolus" or "intravenous push" refers to drug administration into a vein of an animal or human such that the body receives the drug in approximately 15 minutes or less, generally 5 minutes or less.

The term "subcutaneous administration" refers to introduction of an agent under the skin of an animal or human patient, preferable within a pocket between the skin and underlying tissue, by relatively slow, sustained delivery from a drug receptacle. Pinching or drawing the skin up and away from underlying tissue may create the pocket.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is less than approximately 15 minutes; in another aspect, less than 5 minutes, and in still another aspect, less than 60 seconds. In yet even another aspect, administration is within a pocket between the skin and underlying tissue, where the pocket may be created by pinching or drawing the skin up and away from underlying tissue.

The term "therapeutically effective amount" is used to refer to an amount of an active agent that relieves or ameliorates one or more of the symptoms of the disorder being treated. In another aspect, the therapeutically effective amount refers to a target serum concentration that has been shown to be effective in, for example, slowing disease progression. Efficacy can be measured in conventional ways, depending on the condition to be treated.

The terms "treatment" and "therapy" and the like, as used herein, are meant to include therapeutic as well as prophylactic, or suppressive measures for a disease or disorder leading to any clinically desirable or beneficial effect, including but not limited to alleviation or relief of one or more symptoms, regression, slowing or cessation of progression of the disease or disorder. Thus, for example, the term treatment includes the administration of an agent prior to or following the onset of a symptom of a disease or disorder thereby preventing or removing one or more signs of the disease or disorder. As another example, the term includes the administration of an agent after clinical manifestation of the disease to combat the symptoms of the disease. Further, administration of an agent after onset and after clinical symptoms have developed where administration affects clinical parameters of the disease or disorder, such as the degree of tissue injury or the amount or extent of metastasis, whether or not the treatment leads to amelioration of the disease, comprises "treatment" or "therapy" as used herein. Moreover, as long as the compositions of the invention either alone or in combination with another therapeutic agent alleviate or ameliorate at least one symptom of a disorder being treated as compared to that symptom in the absence of use of the anti-IL-23A antibody composition, the result should be considered an effective treatment of the underlying disorder regardless of whether all the symptoms of the disorder are alleviated or not.

The term "package insert" is used to refer to instructions customarily included in commercial packages of therapeutic products, that contain information about the indications, usage, administration, contraindications and/or warnings concerning the use of such therapeutic products.

### Antibodies

The CDRs of selected antibodies used in the context of the present invention are shown in Table 1 and 2. The variable regions of selected antibodies used in the context of the present invention are shown in Table 3 and 4.

**Table 1: LIGHT CHAIN CDR sequences**

| | L-CDR1 | L-CDR2 | L-CDR3 |
|---|---|---|---|
| 6B8 | KASRDVAIAVA (SEQ ID NO:1) | WASTRHT (SEQ ID NO:2) | HQYSSYPFT (SEQ ID NO:3) |

**Table 2: HEAVY CHAIN CDR sequences**

| | **H-CDR1** | **H-CDR2** | **H-CDR3** |
|---|---|---|---|
| **6B8** | **GNTFTDQTIH (SEQ ID NO:4)** | **YIYPRDDSPKYNENFKG (SEQ ID NO:5)** | **PDRSGYAWFIY (SEQ ID NO:6)** |
| **Hu_6B8-2** | **GYTFTDQTIH (SEQ ID NO:7)** | **YIYPRDDSPKYNENFKG (SEQ ID NO:5)** | **PDRSGYAWFIY (SEQ ID NO:6)** |
| **Hu_6B8-5** | **GFTFTDQTIH (SEQ ID NO:8)** | **YIYPRDDSPKYNENFKG (SEQ ID NO:5)** | **PDRSGYAWFIY (SEQ ID NO:6)** |
| **Hu_6B8-36/65** | **GGTFTDQTIH (SEQ ID NO:9)** | **YIYPRDDSPKYNENFKG (SEQ ID NO:5)** | **PDRSGYAWFIY (SEQ ID NO:6)** |

**Table 3: Humanized 6B8-VK Sequences**

| | |
|---|---|
| 6B8CVK-65 | DIQMTQSPSSLSASVGDRVTITCKASRDVAIAVAWYQQKPGKVPKLL LFWASTRHTGVPDRFSGSGSGTDFTLTISSLQPEDLADYYCHQYSSY PFTFGQGTKLEIK (SEQ ID NO:10) |
| 6B8CVK-66 | DIQMTQSPSSLSASVGDRVTITCKASRDVAIAVAWYQQKPGKVPKLL IYWASTRHTGVPSRFSGSGSRTDFTLTISSLQPEDVADYFCHQYSSY PFTFGSGTKLEIK (SEQ ID NO:11) |
| 6B8CVK-67 | DIQMTQSPSSLSASVGDRVTITCKASRDVAIAVAWYQQKPGKVPKLL LYWASTRHTGVPSRFSGSGSRTDFTLTISSLQPEDVATYYCHQYSSY PFTFGSGTKLEIK (SEQ ID NO:12) |
| 6B8CVK-78 | DIQMTQSPSSLSASVGDRVTITCKASRDVAIAVAWYQQKPGKVPKLL LFWASTRHTGVPDRFSGSGSRTDFTLTISSLQPEDLADYYCHQYSSY PFTFGSGTKLEIK (SEQ ID NO:13) |

**Table 4: Humanized 6B8-VH Sequence**

| | |
|---|---|
| 6B8CVH-02 | QVQLVQSGAEVKKPGSSVKVSCKASGYTFTDQTIHWMRQAPGQGLEW |
| | IGYIYPRDDSPKYNENFKGKVTITADKSTSTAYMELSSLRSEDTAVY YCAIPDRSGYAWFIYWGQGTLVTVSS (SEQ ID NO:14) |
| 6B8CVH-05 | QVQLVQSGAEVKKPGSSVKVSCKASGFTFTDQTIHWVRQAPGQGLEW MGYIYPRDDSPKYNENFKGKVTLTADKSTSTAYMELSSLRSEDTAVY YCAIPDRSGYAWFIYWGQGTLVTVSS (SEQ ID NO:15) |
| 6B8CVH-36 | QVQLVQSGAEVKKPGSSVKTSCKASGGTFTDQTIHWVRQRPGQGLEW MGYIYPRDDSPKYNENFKGRVTITADKSTSTAYMELSSLRSEDTAVY YCAIPDRSGYAWFIYWGQGTLVTVSS (SEQ ID NO:16) |
| 6B8CVH-65 | QVQLVQSGAEVKKPGSSVKVSCKASGGTFTDQTIHWVRQAPGQGLEW MGYIYPRDDSPKYNENFKGRVTLTADKSTSTAYMELSSLRSEDTAVY FCARPDRSGYAWFIYWGQGTLVTVSS (SEQ ID NO:17) |

Selected combination of humanized light chain and heavy chain variable regions derived from mouse antibody 6B8 resulted in Antibodies A, B, C and D:
Antibody A: 6B8-IgG1KO-2 with IgK-66 (heavy chain variable region 6B8CVH-02 and light chain variable region 6B8CVK-66);
Antibody B: 6B8-IgG1KO-5 with IgK-66 (heavy chain variable region 6B8CVH-05 and light chain variable region 6B8CVK-66);
Antibody C: 6B8-IgG1KO-2 with IgK-65 (heavy chain variable region 6B8CVH-02 and light chain variable region 6B8CVK-65);
Antibody D: 6B8-IgG1KO-5 with IgK-65 (heavy chain variable region 6B8CVH-05 and light chain variable region 6B8CVK-65).
Antibodies A, B, C and D have the heavy and light chain sequences shown in Table 5.

**Table 5: Heavy and Light Chain DNA and Amino Acid Sequences for Antibodies A, B, C, and D**

| | | |
|---|---|---|
| **Antibody A** | IgK light Chain #66 | DIQMTQSPSSLSASVGDRVTITCKASRDVAIAVAWYQ QKPGKVPKLLIYWASTRHTGVPSRFSGSGSRTDFTLT ISSLQPEDVADYFCHQYSSYPFTFGSGTKLEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC (SEQ ID |
| | IgG1 KO Heavy Chain #2 | NO:18) QVQLVQSGAEVKKPGSSVKVSCKASGYTFTDQTIHWM RQAPGQGLEWIGYIYPRDDSPKYNENFKGKVTITADK STSTAYMELSSLRSEDTAVYYCAIPDRSGYAWFIYWG QGTLVTVSSASTKGPSVFPLAPSSKSTSGGTAALGCL VKDYFPEPVTVSWNSGALTSGVHTFPAVLQSSGLYSL SSVVTVPSSSLGTQTYICNVNHKPSNTKVDKRVEPKS CDKTHTCPPCPAPEAAGGPSVFLFPPKPKDTLMISRT PEVTCVVVDVSHEDPEVKFNWYVDGVEVHNAKTKPRE EQYNSTYRVVSVLTVLHQDWLNGKEYKCKVSNKALPA PIEKTISKAKGQPREPQVYTLPPSREEMTKNQVSLTC LVKGFYPSDIAVEWESNGQPENNYKTTPPVLDSDGSF FLYSKLTVDKSRWQQGNVFSCSVMHEALHNHYTQKSL SLSPG (SEQ ID NO:19) |
| | | |
| **Antibody B** | IgK light Chain #66 | ( SEQ ID NO:18) |
| | IgGIKO Heavy Chain #5 | QVQLVQSGAEVKKPGSSVKVSCKASGFTFTDQTIHWV RQAPGQGLEWMGYIYPRDDSPKYNENFKGKVTLTADK STSTAYMELSSLRSEDTAVYYCAIPDRSGYAWFIYWG QGTLVTVSSASTKGPSVFPLAPSSKSTSGGTAALGCL VKDYFPEPVTVSWNSGALTSGVHTFPAVLQSSGLYSL SSVVTVPSSSLGTQTYICNVNHKPSNTKVDKRVEPKS CDKTHTCPPCPAPEAAGGPSVFLFPPKPKDTLMISRT PEVTCVVVDVSHEDPEVKFNWYVDGVEVHNAKTKPRE EQYNSTYRVVSVLTVLHQDWLNGKEYKCKVSNKALPA PIEKTISKAKGQPREPQVYTLPPSREEMTKNQVSLTC LVKGFYPSDIAVEWESNGQPENNYKTTPPVLDSDGSF FLYSKLTVDKSRWQQGNVFSCSVMHEALHNHYTQKSL SLSPG (SEQ ID NO:20) |
| | | |
| **Antibody C** | IgK | DIQMTQSPSSLSASVGDRVTITCKASRDVAIAVAWYQ |
| | light Chain #65 | QKPGKVPKLLLFWASTRHTGVPDRFSGSGSGTDFTLT ISSLQPEDLADYYCHQYSSYPFTFGQGTKLEIKRTVA APSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC (SEQ ID NO:21) |
| | IgGIKO Heavy Chain #2 | (SEQ ID NO:19) |
| | | |
| **Antibody D** | IgK light Chain #65 | (SEQ ID NO:21) |
| | IgG1KO Heavy Chain #5 | (SEQ ID NO:20) |

Light chains and heavy chain variable regions of Antibodies A, B, C, and D are underlined in Table 5 above.

In one embodiment, an anti-IL-23A antibody comprises the light chain sequence of SEQ ID NO:18 and the heavy chain sequence of SEQ ID NO:19. In one embodiment, an anti-IL-23A antibody comprises the light chain sequence of SEQ ID NO:18 and the heavy chain sequence of SEQ ID NO:20. In one embodiment, an anti-IL-23A antibody comprises the light chain sequence of SEQ ID NO:21 and the heavy chain sequence of SEQ ID NO:19. In one embodiment, an anti-IL-23A antibody comprises the light chain sequence of SEQ ID NO:21 and the heavy chain sequence of SEQ ID NO:20.

In one embodiment, an anti-IL-23A antibody consists of the light chain sequence of SEQ ID NO:18 and the heavy chain sequence of SEQ ID NO:19. In one embodiment, an anti-IL-23A antibody consists of the light chain sequence of SEQ ID NO:18 and the heavy chain sequence of SEQ ID NO:20. In one embodiment, an anti-IL-23A antibody consists of the light chain sequence of SEQ ID NO:21 and the heavy chain sequence of SEQ ID NO:19. In one embodiment, an anti-IL-23A antibody consists of the light chain sequence of SEQ ID NO:21 and the heavy chain sequence of SEQ ID NO:20.

In a further embodiment, an anti-IL-23A antibody binds to human IL-23A at an epitope consisting of amino acid residues 108 to 126 and amino acid residues 137 to 151 of SEQ ID NO: 22.

In a further embodiment, an anti-IL-23A antibody competitively binds to human IL-23A with an antibody of the present invention, for example Antibody A, Antibody B, Antibody C or Antibody D described herein. The ability of an antibody to competitively bind to IL-23A can be measured using competitive binding assays known in the art.

In some embodiments, an anti-IL-23A antibody comprises light chain variable region sequences having the amino acid sequence set forth in of SEQ ID NO:10, 11, 12 or 13. In some embodiments, an anti-IL-23A antibody comprises heavy chain variable region sequences having the amino acid sequence set forth in of SEQ ID NO:14, 15, 16 or 17 (see Tables 3 and 4 above). The CDR sequences of these antibodies are shown in Tables 1 and 2. For example, anti-IL-23A antibodies are monoclonal antibodies with the combinations of light chain variable and heavy chain variable regions of SEQ ID NO: 11/14, 11/15, 10/14 or 10/15. Such variable regions can be combined with human constant regions.

### Polynucleotides, Vectors, Host Cells, and Recombinant Methods

Other embodiments encompass isolated polynucleotides that comprise a sequence encoding an anti-IL-23A antibody, vectors, and host cells comprising the polynucleotides, and recombinant techniques for production of the humanized antibody. The isolated polynucleotides can encode any desired form of the anti-IL-23A antibody including, for example, full length monoclonal antibodies, Fab, Fab', F(ab')₂, and Fv fragments.

The polynucleotide(s) that comprise a sequence encoding an anti-IL-23A antibody can be fused to one or more regulatory or control sequence, as known in the art, and can be contained in suitable expression vectors or host cell as known in the art. Each of the polynucleotide molecules encoding the heavy or light chain variable domains can be independently fused to a polynucleotide sequence encoding a constant domain, such as a human constant domain, enabling the production of intact antibodies. Alternatively, polynucleotides, or portions thereof, can be fused together, providing a template for production of a single chain antibody.

For recombinant production, a polynucleotide encoding the antibody is inserted into a replicable vector for cloning (amplification of the DNA) or for expression. Many suitable vectors for expressing the recombinant antibody are available. The vector components generally include, but are not limited to, one or more of the following: a signal sequence, an origin of replication, one or more marker genes, an enhancer element, a promoter, and a transcription termination sequence.

The anti-IL-23A antibodies can also be produced as fusion polypeptides, in which the antibody is fused with a heterologous polypeptide, such as a signal sequence or other polypeptide having a specific cleavage site at the amino terminus of the mature protein or polypeptide. The heterologous signal sequence selected is typically one that is recognized and processed (i.e., cleaved by a signal peptidase) by the host cell. For prokaryotic host cells that do not recognize and process the anti-IL-23A antibody signal sequence, the signal sequence can be substituted by a prokaryotic signal sequence. The signal sequence can be, for example, alkaline phosphatase, penicillinase, lipoprotein, heat-stable enterotoxin II leaders, and the like. For yeast secretion, the native signal sequence can be substituted, for example, with a leader sequence obtained from yeast invertase alpha-factor (including Saccharomyces and Kluyveromyces α-factor leaders), acid phosphatase, C. albicans glucoamylase, or the signal described in WO90/13646. In mammalian cells, mammalian signal sequences as well as viral secretory leaders, for example, the herpes simplex gD signal, can be used. The DNA for such precursor region is ligated in reading frame to DNA encoding the anti-IL-23A antibody.

Expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2-υ. plasmid origin is suitable for yeast, and various viral origins (SV40, polyoma, adenovirus, VSV, and BPV) are useful for cloning vectors in mammalian cells. Generally, the origin of replication component is not needed for mammalian expression vectors (the SV40 origin may typically be used only because it contains the early promoter).

Expression and cloning vectors may contain a gene that encodes a selectable marker to facilitate identification of expression. Typical selectable marker genes encode proteins that confer resistance to antibiotics or other toxins, e.g., ampicillin, neomycin, methotrexate, or tetracycline, or alternatively, are complement auxotrophic deficiencies, or in other alternatives supply specific nutrients that are not present in complex media, e.g., the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid, and hygromycin. Common selectable markers for mammalian cells are those that enable the identification of cells competent to take up a nucleic acid encoding an anti-IL-23A antibody, such as DHFR (dihydrofolate reductase), thymidine kinase, metallothionein-I and -II (such as primate metallothionein genes), adenosine deaminase, ornithine decarboxylase, and the like. Cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., DG44).

Alternatively, host cells (particularly wild-type hosts that contain endogenous DHFR) transformed or co-transformed with DNA sequences encoding an anti-IL-23A antibody, wild-type DHFR protein, and another selectable marker such as aminoglycoside 3'-phosphotransferase (APH), can be selected by cell growth in medium containing a selection agent for the selectable marker such as an aminoglycosidic antibiotic, e.g., kanamycin, neomycin, or G418. See, e.g., U.S. Pat. No. 4,965,199.

Where the recombinant production is performed in a yeast cell as a host cell, the TRP1 gene present in the yeast plasmid YRp7 (Stinchcomb et al., 1979, Nature 282: 39) can be used as a selectable marker. The TRP1 gene provides a selection marker for a mutant strain of yeast lacking the ability to grow in tryptophan, for example, ATCC No. 44076 or PEP4-1 (Jones, 1977, Genetics 85:12). The presence of the trp1 lesion in the yeast host cell genome then provides an effective environment for detecting transformation by growth in the absence of tryptophan. Similarly, Leu2p-deficient yeast strains such as ATCC 20,622 and 38,626 are complemented by known plasmids bearing the LEU2 gene.

In addition, vectors derived from the 1.6 µm circular plasmid pKD1 can be used for transformation of Kluyveromyces yeasts. Alternatively, an expression system for large-scale production of recombinant calf chymosin was reported for K. lactis (Van den Berg, 1990, Bio/Technology 8:135). Stable multi-copy expression vectors for secretion of mature recombinant human serum albumin by industrial strains of Kluyveromyces have also been disclosed (Fleer et al., 1991, Bio/Technology 9:968-975).

Expression and cloning vectors usually contain a promoter that is recognized by the host organism and is operably linked to the nucleic acid molecule encoding an anti-IL-23p19 antibody or polypeptide chain thereof. Promoters suitable for use with prokaryotic hosts include phoA promoter, β-lactamase and lactose promoter systems, alkaline phosphatase, tryptophan (trp) promoter system, and hybrid promoters such as the tac promoter. Other known bacterial promoters are also suitable. Promoters for use in bacterial systems also will contain a Shine-Dalgarno (S.D.) sequence operably linked to the DNA encoding the anti-IL-23A antibody.

Many eukaryotic promoter sequences are known. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Inducible promoters have the additional advantage of transcription controlled by growth conditions. These include yeast promoter regions for alcohol dehydrogenase 2, isocytochrome C, acid phosphatase, derivative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-IL-23A antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a Hindlll E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., 1982, Nature 297:598-601, disclosing expression of human p-interferon cDNA in mouse cells under the control of a thymidine kinase promoter from herpes simplex virus. Alternatively, the Rous sarcoma virus long terminal repeat can be used as the promoter.

Another useful element that can be used in a recombinant expression vector is an enhancer sequence, which is used to increase the transcription of a DNA encoding an anti-IL-23A antibody by higher eukaryotes. Many enhancer sequences are now known from mammalian genes (e.g., globin, elastase, albumin, α-fetoprotein, and insulin). Typically, however, an enhancer from a eukaryotic cell virus is used. Examples include the SV40 enhancer on the late side of the replication origin (bp 100-270), the cytomegalovirus early promoter enhancer, the polyoma enhancer on the late side of the replication origin, and adenovirus enhancers. See also Yaniv, 1982, Nature 297:17-18 for a description of enhancing elements for activation of eukaryotic promoters. The enhancer may be spliced into the vector at a position 5' or 3' to the anti-IL-23A antibody-encoding sequence, but is preferably located at a site 5' from the promoter.

Expression vectors used in eukaryotic host cells (yeast, fungi, insect, plant, animal, human, or nucleated cells from other multicellular organisms) can also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding anti-IL-23A antibody. One useful transcription termination component is the bovine growth hormone polyadenylation region. See WO94/11026 and the expression vector disclosed therein. In some embodiments, humanized anti-IL-23p19 antibodies can be expressed using the CHEF system. (See, e.g., U.S. Pat. No. 5,888,809; the disclosure of which is incorporated by reference herein.)

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, e.g., E. coli, Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41 P disclosed in DD 266,710 published Apr. 12, 1989), Pseudomonas such as P. aeruginosa, and Streptomyces. One preferred E. coli cloning host is E. coli 294 (ATCC 31,446), although other strains such as E. coli B, E. coli X1776 (ATCC 31,537), and E. coli W3110 (ATCC 27,325) are suitable. These examples are illustrative rather than limiting.

In addition to prokaryotes, eukaryotic microbes such as filamentous fungi or yeast are suitable cloning or expression hosts for anti-IL-23A antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e.g., K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastors (EP 183,070); Candida; Trichoderma reesia (EP 244,234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated anti-IL-23A antibody are derived from multicellular organisms. Examples of invertebrate cells include plant and insect cells, including, e.g., numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori (silk worm). A variety of viral strains for transfection are publicly available, e.g., the L-1 variant of Autographa californica NPV and the Bm-5 strain of Bombyx mori NPV, and such viruses may be used, particularly for transfection of Spodoptera frugiperda cells.

Plant cell cultures of cotton, corn, potato, soybean, petunia, tomato, and tobacco can also be utilized as hosts.

In another aspect, expression of anti-IL-23A antibodies is carried out in vertebrate cells. The propagation of vertebrate cells in culture (tissue culture) has become routine procedure and techniques are widely available. Examples of useful mammalian host cell lines are monkey kidney CV1 line transformed by SV40 (COS-7, ATCC CRL 1651), human embryonic kidney line (293 or 293 cells subcloned for growth in suspension culture, (Graham et al., 1977, J. Gen Virol. 36: 59), baby hamster kidney cells (BHK, ATCC CCL 10), Chinese hamster ovary cells/-DHFR1 (CHO, Urlaub et al., 1980, Proc. Natl. Acad. Sci. USA 77: 4216; e.g., DG44), mouse sertoli cells (TM4, Mather, 1980, Biol. Reprod. 23:243-251), monkey kidney cells (CV1 ATCC CCL 70), African green monkey kidney cells (VERO-76, ATCC CRL-1587), human cervical carcinoma cells (HELA, ATCC CCL 2), canine kidney cells (MDCK, ATCC CCL 34), buffalo rat liver cells (BRL 3A, ATCC CRL 1442), human lung cells (W138, ATCC CCL 75), human liver cells (Hep G2, HB 8065), mouse mammary tumor (MMT 060562, ATCC CCL51), TR1 cells (Mather et al., 1982, Annals N.Y. Acad. Sci. 383: 44-68), MRC 5 cells, FS4 cells, and human hepatoma line (Hep G2).

Host cells are transformed with the above-described expression or cloning vectors for anti-IL-23A antibody production and cultured in conventional nutrient media modified as appropriate for inducing promoters, selecting transformants, or amplifying the genes encoding the desired sequences.

The host cells used to produce an anti-IL-23A antibody described herein may be cultured in a variety of media. Commercially available media such as Ham's F10 (Sigma-Aldrich Co., St. Louis, Mo.), Minimal Essential Medium ((MEM), (Sigma-Aldrich Co.), RPMI-1640 (Sigma-Aldrich Co.), and Dulbecco's Modified Eagle's Medium ((DMEM), Sigma-Aldrich Co.) are suitable for culturing the host cells. In addition, any of the media described in one or more of Ham et al., 1979, Meth. Enz. 58: 44, Barnes et al., 1980, Anal. Biochem. 102: 255, U.S. Pat. No. 4,767,704, U.S. Pat. No. 4,657,866, U.S. Pat. No. 4,927,762, U.S. Pat. No. 4,560,655, U.S. Pat. No. 5,122,469, WO 90/103430, and WO 87/00195 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as gentamicin), trace elements (defined as inorganic compounds usually present at final concentrations in the micromolar range), and glucose or an equivalent energy source. Other supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, the cells may be disrupted to release protein as a first step. Particulate debris, either host cells or lysed fragments, can be removed, for example, by centrifugation or ultrafiltration. Carter et al., 1992, Bio/Technology 10:163-167 describes a procedure for isolating antibodies that are secreted to the periplasmic space of E. coli. Briefly, cell paste is thawed in the presence of sodium acetate (pH 3.5), EDTA, and phenylmethylsulfonylfluoride (PMSF) over about 30 minutes. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants. A variety of methods can be used to isolate the antibody from the host cell.

The antibody composition prepared from the cells can be purified using, for example, hydroxylapatite chromatography, gel electrophoresis, dialysis, and affinity chromatography, with affinity chromatography being a typical purification technique. The suitability of protein A as an affinity ligand depends on the species and isotype of any immunoglobulin Fc domain that is present in the antibody. Protein A can be used to purify antibodies that are based on human gamma1, gamma2, or gamma4 heavy chains (see, e.g., Lindmark et al., 1983 J. Immunol. Meth. 62:1-13). Protein G is recommended for all mouse isotypes and for human gamma3 (see, e.g., Guss et al., 1986 EMBO J. 5:1567-1575). A matrix to which an affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H3} domain, the Bakerbond ABX^{™} resin (J. T. Baker, Phillipsburg, N.J.) is useful for purification. Other techniques for protein purification such as fractionation on an ion-exchange column, ethanol precipitation, reverse phase HPLC, chromatography on silica, chromatography on heparin SEPHAROSE^{™} chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, typically performed at low salt concentrations (e.g., from about 0-0.25M salt).

### Therapeutic Uses

In another embodiment, an anti-IL-23A antibody disclosed herein is useful in the treatment of various disorders associated with the expression of IL-23p19 as described herein. In one aspect, a method for treating an IL-23 associated disorder comprises administering a therapeutically effective amount of an anti-IL-23A antibody to a subject in need thereof.

The anti-IL-23A antibody is administered by any suitable means, including parenteral, subcutaneous, intraperitoneal, intrapulmonary, and intranasal, and, if desired for local immunosuppressive treatment, intralesional administration (including perfusing or otherwise contacting the graft with the antibody before transplantation). The anti-IL-23A antibody or agent can be administered, for example, as an infusion or as a bolus. Parenteral infusions include intramuscular, intravenous, intraarterial, intraperitoneal, or subcutaneous administration. In addition, the anti-IL-23A antibody is suitably administered by pulse infusion, particularly with declining doses of the antibody. In one aspect, the dosing is given by injections, most preferably intravenous or subcutaneous injections, depending in part on whether the administration is brief or chronic. In one aspect, the dosing of the anti-IL-23 antibody is given by subcutaneous injections.

For the prevention or treatment of disease, the appropriate dosage of antibody will depend on a variety of factors such as the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

The term "suppression" is used herein in the same context as "amelioration" and "alleviation" to mean a lessening of one or more characteristics of the disease.

The antibody is formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody to be administered will be governed by such considerations.

The antibody may optionally be formulated with one or more agents currently used to prevent or treat the disorder in question. The effective amount of such other agents depends on the amount of anti-IL-23A antibody present in the formulation, the type of disorder or treatment, and other factors discussed above.

### IL-23-Associated Disorders

The anti-IL-23p19 antibodies or agents are useful for treating or preventing an immunological disorder characterized by abnormal expression of IL-23, e.g., by inappropriate activation of immune cells (e.g., lymphocytes or dendritic cells). Such abnormal expression of IL-23 can be due to, for example, increased IL-23 protein levels.

Immunological diseases that are characterized by inappropriate activation of immune cells and that can be treated or prevented by the methods described herein can be classified, for example, by the type(s) of hypersensitivity reaction(s) that underlie the disorder. These reactions are typically classified into four types: anaphylactic reactions, cytotoxic (cytolytic) reactions, immune complex reactions, or cell-mediated immunity (CMI) reactions (also referred to as delayed-type hypersensitivity (DTH) reactions). (See, e.g., Fundamental Immunology (William E. Paul ed., Raven Press, N.Y., 3rd ed. 1993). Immunological diseases include inflammatory diseases and autoimmune diseases.

Examples of immunological diseases include the following: psoriasis, inflammatory bowel disease, for example ulcerative colitis or Crohn's disease, and spondyloarthritis, for example ankylosing spondylitis, non-radiographic axial spondyloarthritis, peripheral spondyloarthritis or psoriatic arthritis.

In one aspect, in the context of the present invention, the immunological disease is Crohn's Disease, for example moderately to severely active Crohn's Disease. In one aspect, in the context of the present invention, a patient is naive to, or was previously treated with anti-TNF therapy. In one aspect, in the context of a method of the present invention a patient was previously treated with one, two, three or more TNF antagonist(s). In one embodiment, the patient is a patient who had an inadequate response with, lost response, or was intolerant to a TNF antagonist. In one aspect, a patient to be treated by a method according ot the present invention has a CDAI score of 220-450.

Disease severity for Crohn's Disease is for example measured using the Crohn's Disease Activity Index (CDAI). CDAI is a composite score used to quantify symptoms of patients with Crohn's Disease. In one aspect, the index consists of eight factors added together after adjusting for a predefined weighting factor (see below in Table B). CDAI scores range from 0 to 600. Index values of 150 and below are associated with quiescent disease; values above 150 are associated with active disease, and values above 450 are seen with extremely severe disease.

**Table B. Format for calculation of the CDAI**

| **Clinical or laboratory variable** | **Weighting factor** |
|---|---|
| Number of liquid or soft stools each day for 7 days | x2 |
| Abdominal pain (graded from 0 to 3 on severity) each day for 7 days | ×5 |
| General wellbeing, subjectively assessed from 0 (well) to 4 (terrible) each day for 7 days | ×7 |
| Presence of complications | x20 |
| Taking Lomotil or opiates for diarrhoea | x30 |
| Presence of an abdominal mass (0 as none, 2 as questionable, 5 as definite) | x10 |
| Haematocrit of <0.47 in men and <0.42 in women | ×6 |
| Percentage deviation from standard weight | x1 |

At the mucosal level, the extent of disease is for example graded following ileocolonoscopy according to the Crohn's Disease Endoscopic Index of Severity (CDEIS). In one aspect, CDEIS is a validated scoring system in which six endoscopic variables (presence of deep ulcers, superficial ulcers, non-ulcerated stenosis, ulcerated stenosis, proportion of ulcerated surface, and proportion of surface affected by disease) are assessed in each of the five segments: rectum, sigmoid and left colon, transverse colon, right colon, and ileum. For these segments, the percentage of ulcerated colonic surface and the percentage of surface affected by any Crohn's disease lesion are indicated on a 10 cm visual analogue scale. CDEIS scores range from 0 to 44 and higher scores indicate more severe disease.

Other evaluations of the disease are for example described in the Examples hereinbelow. In one aspect, CDAI or CDEIS, or both, or any one of the evaluations described in the Examples hereinbelow, is/are used to assess the efficacy of an anti-IL-23A antibody, for example Antibody A, Antibody B, Antibody C or Antibody D, in the treatment of Crohn's Disease, for example moderately to severely active Crohn's Disease.

For example, a patient is evaluated for clinical remission, for example defined as a CDAI score of < 150.

For example, a patient is evaluated for clinical response, for example defined by either a CDAI score of < 150 or a CDAI reduction from baseline of at least 100 points.

For example, a patient is evaluated for endoscopic remission, for example defined as CDEIS ≤ 4. For patients with initial isolated ileitis, endoscopic remission is for example defined as CDEIS ≤ 2.

For example, a patient is evaluated for endoscopic response, for example defined as a >50% CDEIS reduction from baseline.

For example, a patient is evaluated for deep remission, for example defined as reaching clinical remission (CDAI score <150) and/or endoscopic remission (CDEIS ≤ 4). In one, aspect, for a patient with initial isolated ileitis endoscopic remission is defined by CDEIS ≤ 2. For example, deep remission is defined as achieving clinical remission and endoscopic remission.

In one aspect, the Patient reported outcome (PRO) of a patient is assessed, for example using a PRO-2 score. In one aspect, PRO-2 remission is assessed, for example as defined by a PRO-2 score of ≤75. In one aspect, PRO-2 response is assessed, for example as defined as a decrease from baseline of 50 points or more.

In one aspect, PRO-2 includes only the two CDAI items stool frequency and abdominal pain. In one aspect, the PRO-2 is calculated based on the sum of the weighted patient-reported subscores of CDAI for liquid or soft stool frequency plus abdominal pain in the 7 days prior to the study visit. PRO-2 is calculated by adding the values of the summed stool frequency scores multiplied by 2 plus the summed abdominal pain scores multiplied by 5.

In one aspect, Health-related quality of life (HRQoL) is assessed by asking patients to complete the 32 questions of the Inflammatory Bowel Disease Questionnaire (IBDQ), which is a tool to scale the impact of bowel-related symptoms, systemic complaints, social functions, and emotional status on HRQoL, with higher scores indicating better HRQoL. A mean change of 16 points is considered clinically meaningful for this instrument.

In one aspect, in the context of the present invention, the immunological disease is ulcerative colitis. In one aspect, an anti-IL-23A antibody, for example Antibody A, Antibody B, Antibody C or Antibody D, is used for the treatment of patients with moderately to severely active ulcerative colitis, for example patients who have had an inadequate response with, lost response to, or were intolerant to conventional therapy or tumour necrosis factor-alpha (TNFα) antagonist. For example, the treatment is by inducing and maintaining clinical remission, by inducing and maintaining clinical response, by improving endoscopic appearance of the mucosa or by achieving corticosteroid-free remission.

### Pharmaceutical Compositions and Administration Thereof

A composition comprising an anti-IL-23A antibody can be administered to a subject having or at risk of having an immunological disorder. The term "subject" as used herein means any mammalian patient to which an anti-IL-23A antibody can be administered, including, e.g., humans and non-human mammals, such as primates, rodents, and dogs. Subjects specifically intended for treatment using the methods described herein include humans. The antibodies can be administered either alone or in combination with other compositions in the prevention or treatment of the immunological disorder.

Anti-IL-23A antibodies for use in such pharmaceutical compositions are described herein, for example Antibody A, Antibody B, Antibody C or Antibody D.

Various delivery systems are known and can be used to administer the anti-IL-23A antibody. Methods of introduction include but are not limited to intradermal, intramuscular, intraperitoneal, intravenous, subcutaneous, intranasal, epidural, and oral routes. The anti-IL-23A antibody can be administered, for example by infusion, bolus or injection, and can be administered together with other biologically active agents such as chemotherapeutic agents. Administration can be systemic or local. In one embodiment, the administration is by subcutaneous injection. Formulations for such injections may be prepared in for example prefilled syringes that may be administered once every other week.

In specific embodiments, the anti-IL-23A antibody is administered by injection, by means of a catheter, by means of a suppository, or by means of an implant, the implant being of a porous, non-porous, or gelatinous material, including a membrane, such as a sialastic membrane, or a fiber. Typically, when administering the composition, materials to which the anti-IL-23A antibody or agent does not absorb are used.

In other embodiments, the anti-IL-23A antibody is delivered in a controlled release system. In one embodiment, a pump may be used (see, e.g., Langer, 1990, Science 249:1527-1533; Sefton, 1989, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used. (See, e.g., Medical Applications of Controlled Release (Langer and Wise eds., CRC Press, Boca Raton, Fla., 1974); Controlled Drug Bioavailability, Drug Product Design and Performance (Smolen and Ball eds., Wiley, New York, 1984); Ranger and Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.) Other controlled release systems are discussed, for example, in Langer, supra.

An anti-IL-23p19 antibody is typically administered as pharmaceutical compositions comprising a therapeutically effective amount of the antibody and one or more pharmaceutically compatible ingredients.

In typical embodiments, the pharmaceutical composition is formulated in accordance with routine procedures as a pharmaceutical composition adapted for intravenous or subcutaneous administration to human beings. Typically, compositions for administration by injection are solutions in sterile isotonic aqueous buffer. Where necessary, the pharmaceutical can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the pharmaceutical is to be administered by infusion, it can be dispensed with an infusion bottle containing sterile pharmaceutical grade water or saline. Where the pharmaceutical is administered by injection, an ampoule of sterile water for injection or saline can be provided so that the ingredients can be mixed prior to administration.

Further, the pharmaceutical composition can be provided as a pharmaceutical kit comprising (a) a container containing an anti-IL-23A antibody in lyophilized form and (b) a second container containing a pharmaceutically acceptable diluent (e.g., sterile water) for injection. The pharmaceutically acceptable diluent can be used for reconstitution or dilution of the lyophilized anti-IL-23A antibody. Optionally associated with such container(s) can be a notice in the form prescribed by a governmental agency regulating the manufacture, use or sale of pharmaceuticals or biological products, which notice reflects approval by the agency of manufacture, use or sale for human administration.

Examples of pharmaceutical compositions used in the context of the present invention are disclosed in Example 2 hereinbelow.

The invention is further described in the following examples, which are not intended to limit the scope of the invention.

### Examples

### Example 1: Clinical Study

This trial is a proof of concept, multi-center, randomized, double-blind, placebo-controlled, parallel-group phase 2 dose-ranging study of Antibody A in patients with moderately to severely active CD.

The trial consist of a screening period of up to a maximum of 4 weeks, a 12-week blinded intravenous therapy period (Period 1), a 14-week open label intravenous therapy /wash-out period (Period 2), a 26-week subcutaneous therapy period (Period 3) and a 15-week follow up period.

Approximately 240 patients are screened and approximately 120 patients with moderate to severe CD and mucosal ulcers detected on ileocolonoscopy are randomized in a ratio of 1:1:1 to one of the 3 following treatment groups in Period 1:
- Group 1: Placebo IV (n=40)
- Group 2: Antibody A 200 mg IV (n=40)
- Group 3: Antibody A 600 mg IV (n=40)

Randomization is stratified according to previous experience with anti-TNF therapy (naive vs. experienced). Safety and efficacy evaluations are performed through the end of the study. The end of study is defined as the date the last patient completes the last follow up visit.

Each treatment group receives the corresponding dose of Antibody A or placebo by IV infusion at week 0, week 4, and week 8. At week 12, patients are evaluated for deep remission, defined as reaching clinical remission (CDAI score <150) and endoscopic remission (CDEIS ≤ 4) confirmed by central independent reviewer(s). For patients with initial isolated ileitis endoscopic remission is defined by CDEIS ≤ 2.

Treatment in Period 2 is determined by the outcome at week 12.
- Patients who are in deep remission at week 12 stop medication, and enter a wash-out period until week 26.

In case of a disease flare during this period (including visit E1), defined as an increase of CDAI score ≥ 70 points compared to week 12 and a CDAI score of 220 or greater, the investigator performs an ileocolonoscopy within 2 weeks;
∘ If the CDEIS score is ≤ 4 (in patients with initial ileitis ≤ 2), patients continue on wash-out until week 26.
∘ If the CDEIS score is > 4 (in patients with initial ileitis >2), patients receive open-label intravenous induction therapy (3 doses separated by 4 week intervals) with 600 mg of Antibody A, as described in Figure 1.

- Patients who do not achieve deep remission at week 12 receive open label intravenous induction therapy (3 doses separated by 4-week intervals) with 600 mg of Antibody A, as described in Figure 1.

Patients who are in clinical remission at visit E1, irrespective of their week 12 outcome and period 2 treatment, enter Period 3 (open label subcutaneous period) and receive 4 injections of Antibody A (180 mg SC) separated by 8-week intervals.

Ileocolonoscopy is performed for all patients at screening, week 12 and 52 to evaluate endoscopic response/remission, and to provide mucosal biopsy specimens for molecular pharmacodynamic assessments pre and post treatment. Patients who experienced flare after achieving deep remission at week 12 are also required to undergo ileocolonoscopy and receive open-label re-induction therapy only if their CDEIS is >4 (in patients with initial ileitis >2) confirmed by central independent reviewer(s). All colonoscopies are videotaped with the use of a standard protocol and interpreted by an independent reviewer(s), who is (are) unaware of study-group assignments and the timing of the procedure. Patients participating in the study consent to undergo up to 4 colonoscopies.

Criteria for efficacy are as follow:
Primary efficacy endpoint is:
   Clinical remission, for example at week 12, defined as a CDAI score of < 150.
Secondary efficacy endpoints are:
   - Clinical response, for example at week 12, defined by either a CDAI score of < 150 or a CDAI reduction from baseline of at least 100 points.
   - PRO response, for example at week 12, defined by either a PRO-2 score of <8 or a reduction from baseline of at least 8 points (PRO-2: Patient reported outcome-2).
   - CDEIS remission, defined as a score of 4 or less, for example at week 12 (for patients with initial isolated ileitis a score of 2 or less).
   - CDEIS response, defined as a score of 7 or less, for example at week 12 (for patients with initial isolated ileitis >50% reduction from baseline).
   - Change in SES-CD score, for example at week 12.
   - Mucosal healing, defined as the absence of mucosal ulceration, for example at week 12.
   - Deep remission, defined as clinical remission and endoscopic remission (CDEIS), for example at week 12. Change from baseline in CDAI scores by visit.
Other efficacy endpoints are:
   - Change from baseline in CDAI scores by visit.
   - Change from baseline in PRO-2 scores by visit.
   - Reduction of 75% in CDEIS scores from baseline, for example at week 12.
   - Reduction of 75% in SES-CD scores from baseline, for example at week 12.
   - Time to flare for those who achieve deep remission and stop medication, for example at Week 12.
   - Time to flare for those who achieve clinical remission, for example at Week 26.
   - Change from baseline in stool frequency by visit based on patient diary.
   - Stool consistency by visit based on patient diary.
   - Change from baseline in abdominal pain scores by visit based on the patient diary and scored, on a numeric rating scale from 0 (no pain) to 10 (worst possible pain).
   - Change from baseline in IBDQ scores by visit.
   - Change from baseline in CRP (C-reactive protein), calprotectin and lactoferrin profile by visit.
   - Sustained clinical remission after corticosteroid withdrawal (from week 12).
   - Reduction in the number of draining fistulas in patients with draining fistulas at baseline.
   - PRO-2 remission, for example at week 204/206/216, defined by a PRO-2 score of <75.
   - PRO-2 response, for example at week 204/206/216, defined as a decrease from baseline of 50 points or more.
   - Change in CDEIS by visit.
   - Change in SES-CD by visit.
   - CDEIS percentage change from baseline by visit.
   - SES-CD percentage change from baseline by visit.

### Methods

The study comprised three treatment periods: a 12-week double-blinded intravenous (iv) induction period, a 14-week open-label iv re-induction/wash-out period (i.e. re-induction at weeks 14-26 or wash-out at weeks 12-26 for patients who do not undergo re-induction), and a 26-week subcutaneous maintenance period. In the induction period, patients (N=121), with clinically active disease (CD Activity Index [CDAI] score ≥220) confirmed by endoscopy (CD Endoscopic Index of Severity [CDEIS] score ≥7; ≥4 for patients with isolated ileitis), who failed either a TNF antagonist or conventional CD therapy, were randomly assigned to receive either Antibody A (200 mg or 600 mg) or placebo, at Weeks 0, 4, and 8. The primary endpoint was clinical remission (CDAI <150) at Week 12. Secondary endpoints at Week 12 included clinical response, endoscopic remission/response and deep remission.

Eligible patients were aged 18-75 years. They had had a diagnosis of CD for at least 3 months and at screening had moderate-to-severe CD, defined as a CD Activity Index (CDAI) of 220-450, with mucosal ulcers in the ileum and/or colon, and a CD Endoscopic Index of Severity (CDEIS) ≥7 (≥4 for patients with isolated ileitis) on ileocolonoscopy scored by a blinded central reader. Patients either naive or experienced to one or more tumor necrosis factor (TNF) antagonists were included (Table C). Some patients had an inadequate response with, lost response, or were intolerant to a TNF antagonist (Table C). Patients previously treated with ustekinumab were excluded, as were patients who had received any biologic agent within 8 weeks or 5 half-lives of the biologic prior to randomization, i.e. prior to the first administration of study agent.

Differences between Antibody A and placebo were analyzed using appropriate tests for pairwise comparisons of binomial data. Results for the induction period are reported.

**Table C. Patients with previous TNF antagonist and outcome**

| | | Placebo | Antibody A 200mg iv | Antibody A 600mg iv | Total |
|---|---|---|---|---|---|
| | | N (%) | N (%) | N (%) | N (%) |
| Number of Patients | | 39 (100.0) | 41 (100.0) | 41 (100.0) | 121 (100.0) |
| Prior TNF antagonist use, n(%) | | 39(100.0) | 41 (100.0) | 41 (100.0) | 121 (100.0) |
| | 1 | 12 ( 30.8) | 9 ( 22.0) | 9 (22.0) | 30 ( 24.8) |
| | 2 | 20 ( 51.3) | 23 ( 56.1) | 24 ( 58.5) | 67 ( 55.4) |
| | >=3 | 5 ( 12.8) | 7(17.1) | 4(9.8) | 16 ( 13.2) |
| | Missing | 2 ( 5.1) | 2 ( 4.9) | 4 ( 9.8) | 8 ( 6.6) |
| Outcome of >=1 previous TNF antagonist, n(%) | | 35 ( 89.7) | 37 ( 90.2) | 36 ( 87.8) | 108 ( 89.3) |
| | Inadequate Response | 10 ( 25.6) | 16 (39.0) | 11 (26.8) | 37 ( 30.6) |
| | Loss of response | 21 ( 53.8) | 13(31.7) | 17 ( 41.5) | 51 (42.1) |
| | Unacceptable adverse events | 1 (2.6) | 2 ( 4.9) | 5 ( 12.2) | 8 ( 6.6) |
| | Others | 2 ( 5.1) | 3 ( 7.3) | 1 (2.4) | 6 ( 5.0) |
| Unknown | | 1 (2.6) | 3 ( 7.3) | 2 ( 4.9) | 6 ( 5.0) |

### Results

Baseline demographics and disease characteristics were similar between study arms. In total, there were 47 males and 74 females, with a mean age of 38.1 years and mean CDAI and CDEIS scores of 306.8 and 13.4; 94.2% of patients had previously been exposed to ≥1 TNF antagonists. At Week 12, clinical remission was achieved by 24.4% and 36.6% of patients with 200 mg and 600 mg Antibody A, respectively, compared with 15.4% of patients with placebo (p=0.308 and p=0.025) (Table 6); clinical response rates were 36.6% and 41.5% in the 200 mg and 600 mg Antibody A arms, compared with 20.5% in the placebo group (p=0.103 and p=0.037). Endoscopic remission was achieved by 14.6% and 19.5% of patients with 200 mg and 600 mg Antibody A, compared with 2.6% of patients with placebo (p=0.056 and p=0.017); endoscopic response was achieved by 26.8% and 36.6% of patients with 200 mg and 600 mg Antibody A, compared with 12.8% of patients with placebo (p=0.117 and p=0.014). Deep remission was achieved by 2.4% and 12.2% of patients with 200 mg and 600 mg Antibody A, compared with 0.0% for placebo (p=1.0 and p=0.062). Mucosal healing was detected in 3.0%, 2.9% and 7.5% of patients with placebo, 200 mg and 600 mg Antibody A. Adverse events (AEs) were similar between Antibody A and placebo with no dose-related increase in AEs. Fewer severe and serious AEs were reported in the 600 mg Antibody A arm. Results are shown in Tables 6 and 7. Tables 6 and 7 include pooled values for Antibody A.

The clinical remission and clinical response of Antibody A over 12 weeks are also shown in Figure 2. In Figure 2, the following are depicted from left to right for week 4, week 8 and week 12: Placebo, 200 mg Antibody A, 600 mg Antibody A, and Pooled Antibody A.

Figure 2A, clinical remission over time:
Week 4: Placebo (3 patients/7.7% with clinical remission), 200 mg Antibody A (4 patients/9.8% with clinical remission), 600 mg Antibody A (8 patients/19.5% with clinical remission), Pooled Antibody A (12 patients/14.6% with clinical remission).
Week 8: Placebo (1 patients/2.6% with clinical remission), 200 mg Antibody A (7 patients/17.1% with clinical remission), 600 mg Antibody A (10 patients/24.4% with clinical remission), Pooled Antibody A (17 patients/20.7% with clinical remission). Week 12: Placebo (6 patients/15.4% with clinical remission), 200 mg Antibody A (10 patients/24.4% with clinical remission), 600 mg Antibody A (15 patients/36.6% with clinical remission), Pooled Antibody A (25 patients/30.5% with clinical remission).

Figure 2B, clinical response over time:
Week 4: Placebo (6 patients/15.4% with clinical response), 200 mg Antibody A (10 patients/24.4% with clinical response), 600 mg Antibody A (13 patients/31.7% with clinical response), Pooled Antibody A (23 patients/28.0% with clinical response).
Week 8: Placebo (5 patients/12.8% with clinical response), 200 mg Antibody A (13 patients/31.7% with clinical response), 600 mg Antibody A (13 patients/31.7% with clinical response), Pooled Antibody A (26 patients/31.7% with clinical response).
Week 12: Placebo (8 patients/20.5% with clinical response), 200 mg Antibody A (15 patients/36.6% with clinical response), 600 mg Antibody A (17 patients/41.5% with clinical response), Pooled Antibody A (32 patients/39.0% with clinical response).

Median CDAIs over time are shown in Figure 3A and Table 8A. PRO-2 response at week 12 is shown in Table 8B, PRO-2 remission at week 12 is shown in Table 8C.

### Safety

There was no dose-related increase in any of the AEs reported with Antibody A (Table 11). The most frequent AEs were related to the gastrointestinal tract. The incidence of severe AEs was higher in the placebo group than the Antibody A groups (23%, 15%, and 7%, respectively). AEs leading to discontinuation were reported for 15%, 12%, and 2% of patients in the placebo, 200 mg, and 600 mg Antibody A arms (Table 11). SAEs were experienced in 31%, 22%, and 7% of patients in the placebo, 200 mg, and 600 mg Antibody A groups, respectively. The most common SAE was worsening of underlying disease. No deaths occurred, while serious infections were reported in three (abdominal, anal and rectal abscess, and pneumonia), one (pneumonia), and two patients (osteomyelitis and anal abscess) in the placebo, 200 mg, and 600 mg Antibody A arms. Infusion-related reactions were mild or moderate and reported in 5%, 2%, and 2% of patients in the placebo, 200 mg, and 600 mg Antibody A arms.

Treatment-emergent anti-drug antibodies (ADAs) were detected in 4% of patients receiving Antibody A (3 of 76 patients). ADA titer values were low (≤8) and there were no neutralizing antibodies detected. Pre-existing ADAs were observed in five patients in the Antibody A dose groups and three patients in the placebo group

### Conclusions

In patients with active CD, selective blockade of IL-23 with Antibody A was more effective than placebo for inducing clinical and endoscopic remission at 12 weeks and was well tolerated.

**Table 6. Efficacy endpoints at Week 12**

| | **Placebo (N=39)** | **Antibody A** | | |
|---|---|---|---|---|
| | | **200 mg (N=41)** | **600 mg (N=41)** | **Pooled 200 mg + 600 mg (N=82)** |
| Clinical remission - n/N (%) | | | | |
| Difference vs placebo | 6/39 (15.4) | 10/41 (24.4) 9.0 | 15/41 (36.6) 20.9 | 25/82 (30.5) 15.1 |
| p-value | n/a | 0.308 | 0.025 | 0.049 |
| Clinical response - n/N (%) | | | | |
| Difference vs placebo | 8/39 (20.5) | 15/41 (36.6) 16.0 | 17/41 (41.5) 20.3 | 32/82 (39.0) 18.4 |
| p-value | n/a | 0.103 | 0.037 | 0.027 |
| Endoscopic remission- n/N (%) | | | | |
| Difference vs placebo | 1/39 (2.6) | 6/41 (14.6) 12.0 | 8/41 (19.5) 16.9 | 14/82 (17.1) 14.5 |
| p-value | n/a | 0.056 | 0.017 | 0.024 |
| Endoscopic response - n/N (%) | | | | |
| Difference vs placebo | 5/39 (12.8) | 11/41 (26.8) 14.0 | 15/41 (36.6) 23.8 | 26/82 (31.7) 18.9 |
| p-value | n/a | 0.117 | 0.014 | 0.026 |
| Deep remission - n/N (%) | | | | |
| Difference vs placebo | 0/39(0.0) | 1/41 (2.4) 2.4 | 5/41 (12.2) 12.2 | 6/82 (7.3) 7.3 |
| p-value | n/a | 1.000 | 0.062 | 0.182 |
| Clinical remission is defined as a CDAI score <150. Clinical response is either a CDAI score <150 or a CDAI reduction from baseline of ≥100 points. Endoscopic remission is a CDEIS score of ≤4; for patients with initial isolated ileitis a score of ≤2. Endoscopic response is a score of 7 or less; for patients with initial isolated ileitis a >50% reduction in CDEIS score from baseline. Deep remission is clinical | | | | |
| remission and endoscopic remission. Full analysis set was used for this analysis, using last observation carried forward for missing values and stratified Cochran-Mantel-Haenszel tests for clinical endpoints; for endoscopic endpoints, non-response imputation was used for missing values and analyzed with Pearson'schi-squared test, using Fisher's exact test for deep remission. CDAI, Crohn's Disease Activity Index; CDEIS, Crohn's Disease Endoscopic Index of Severity. chi- | | | | |

**Table 7. Efficacy endpoints at Week 12**

| Table 7 shows the efficacy endpoints at week 12 with a refined statistical analysis ( also Table 6). Mucosal healing is also shown in Table 7. see | | | | |
|---|---|---|---|---|
| | **Placebo (N=39)** | **Antibody A** | | |
| | | **200 mg (N=41)** | **600 mg (N=41)** | **Pooled (N=82)** |
| **Clinical remission** - n (%) | 6 (15.4) | 10(24.4) | 15 (36.6) | 25 (30.5) |
| 95% confidence interval | 5.9, 30.5 | 12.4, 40.3 | 22.1, 53.1 | 20.8, 41.6 |
| Difference vs. placebo | | 9.0 | 20.9 | 15.1 |
| 95% confidence interval | | -8.3, 26.2 | 2.6, 39.2 | 0.1, 30.1 |
| P-value | | 0.31 | 0.025 | 0.049 |
| **Clinical response** - n (%) | 8 (20.5) | 15 (36.6) | 17 (41.5) | 32 (39.0) |
| 95% confidence interval | 9.3, 36.5 | 22.1, 53.1 | 26.3, 57.9 | 28.4, 50.4 |
| Difference vs. placebo | | 16.0 | 20.3 | 18.4 |
| 95% confidence interval | | -3.2, 35.2 | 1.3, 39.4 | 2.1, 34.8 |
| P-value | | 0.10 | 0.037 | 0.027 |
| **Endoscopic remission** - n (%) | 1 (2.6) | 6 (14.6) | 8 (19.5) | 14 (17.1) |
| | 0.1, 13.5 | 5.6, 29.2 | 8.8, 34.9 | 9.7, 27.0 |
| 95% confidence interval | | 12.1 | 16.8 | 14.5 |
| Difference vs. placebo | | 0.8, 23.4 | 3.9, 29.7 | 5.5, 23.5 |
| 95% confidence interval P-value | | 0.036 | 0.011 | 0.002 |
| **Endoscopic response** - n (%) | 5 (12.8) | 11 (26.8) | 15 (36.6) | 26 (31.7) |
| 95% confidence interval | 4.3, 27.4 | 14.2, 42.9 | 22.1, 53.1 | 21.9, 42.9 |
| Difference vs. placebo | | 14.1 | 23.5 | 18.7 |
| 95% confidence interval | | -2.8, 30.9 | 5.5, 41.5 | 4.4, 33.0 |
| P-value | | 0.10 | 0.011 | 0.010 |
| **Mucosal healing** - n (%) | 1 (2.6) | 1 (2.4) | 3 (7.3) | 4 (4.9) |
| 95% confidence interval | 0.1, 13.5 | 0.1, 12.9 | 1.5, 19.9 | 1.3, 12.0 |
| Difference vs. placebo | | -0.1 | 4.9 | 2.4 |
| 95% confidence interval | | -7.0, 6.7 | -4.6, 14.3 | -4.5,9.2 |
| P-value | | 0.97 | 0.31 | 0.50 |
| **Deep remission** - n (%) | 0 | 1 (2.4) | 5 (12.2) | 6 (7.3) |
| 95% confidence interval | 0.0, 9.0 | 0.1, 12.9 | 4.1, 26.2 | 2.7, 15.2 |
| Difference vs. placebo | | 2.4 | 12.4 | 7.4 |
| 95% confidence interval | | -2.3, 7.1 | 2.3, 22.5 | 1.7, 13.0 |
| P-value | | 0.31 | 0.016 | 0.011 |

Clinical remission is defined as a CDAI score <150. Clinical response is either a CDAI score <150 or a CDAI reduction of ≥100 from baseline. Endoscopic remission is a CDEIS score of ≤4 at Week 12 (≤2 for patients with initial isolated ileitis). Endoscopic response is a >50% reduction in CDEIS score from baseline to Week 12. Mucosal healing is defined as the absence of mucosal ulceration. Deep remission is clinical remission and endoscopic remission. Full analysis set was used for this analysis, using non-response imputation for missing values and stratified Cochran-Mantel-Haenszel tests.

CDAI, Crohn's Disease Activity Index; CDEIS, Crohn's Disease Endoscopic Index of Severity; SD, standard deviation.

### Biomarkers

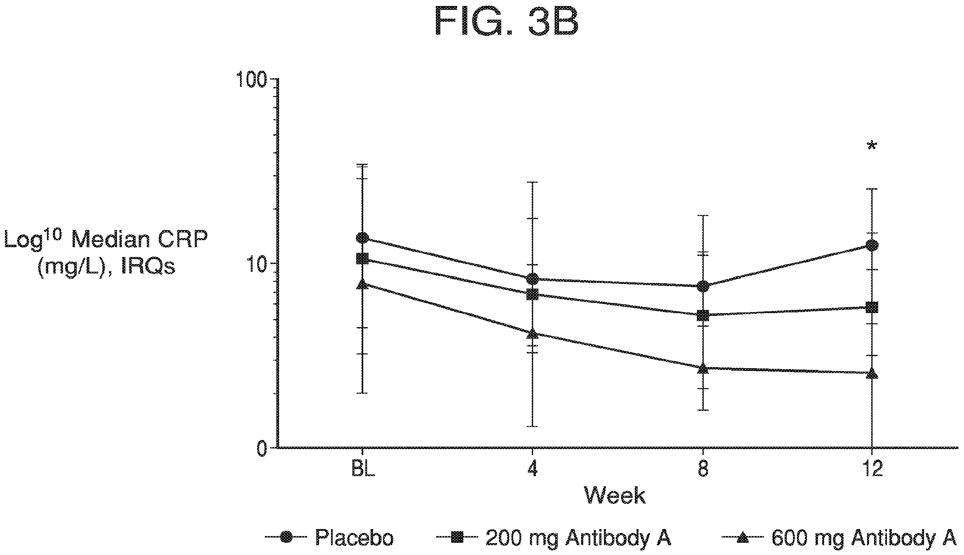

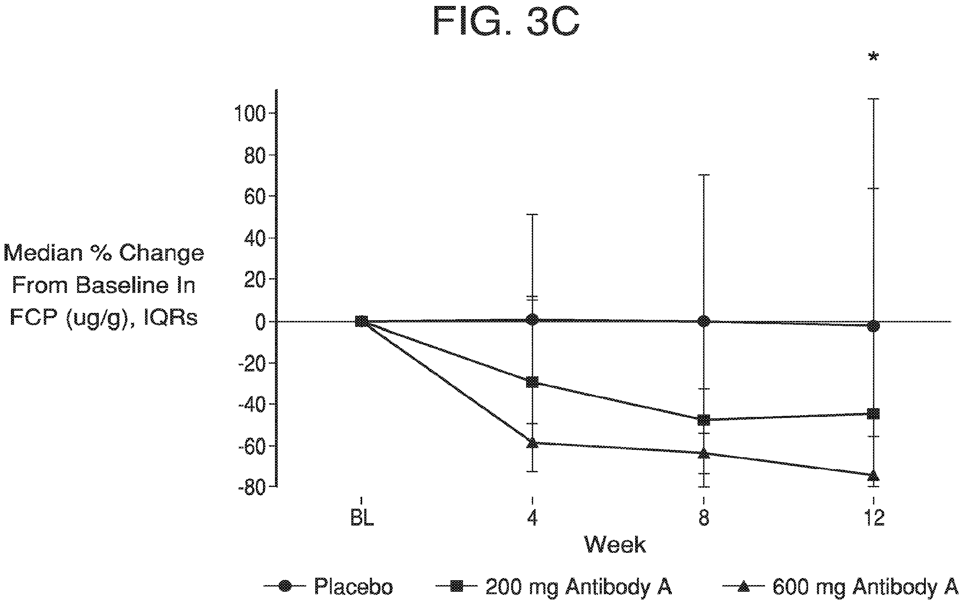

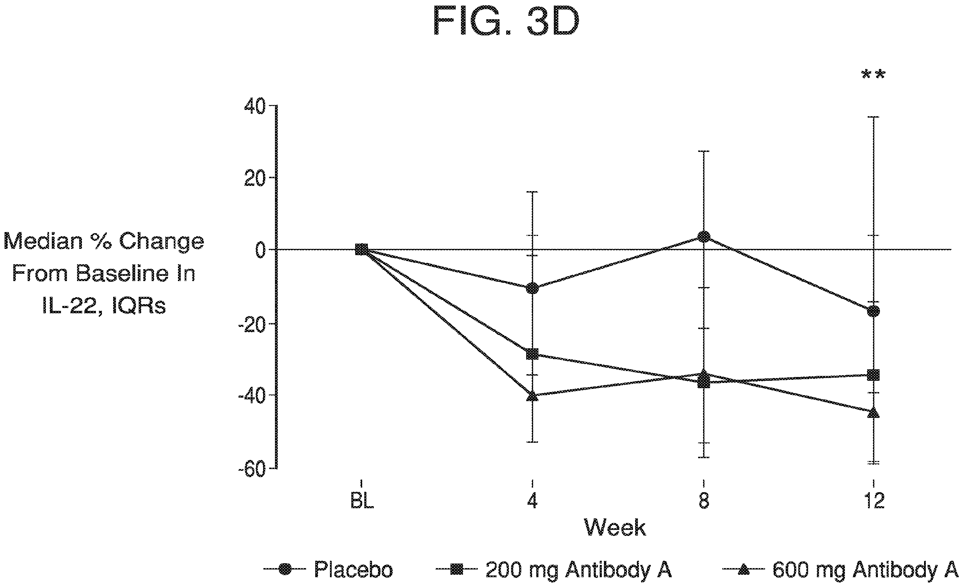

Median CRP concentrations declined over time in both Antibody A arms compared with placebo and were significantly reduced compared with placebo at Week 12 (P<0.001; Figure 3B). Treatment with 600 mg Antibody A significantly decreased FCP levels (baseline to Week 12) compared with placebo (P<0.001; Figure 3C). In addition, significantly greater decreases in plasma IL-22 levels (baseline to Week 12) were observed with 600 mg Antibody A versus placebo (P=0.018; Figure 3D). IL-22 levels were measured in patient plasma using the the Erenna^{®} SMC^{™} IL-22 Immunoassay - a quasi-quantitative fluorescent sandwich immunoassay technique. FCP was measured in feces using an enzyme immunoassay by Buhlmann Laboratories AG and tested by Covance Central laboratories.

Table 8A also shows median CRP (mg/L) over time, median FCP (µg/g) over time and median % change in IL-22 over time (BL: baseline, IQR:interquartile range).

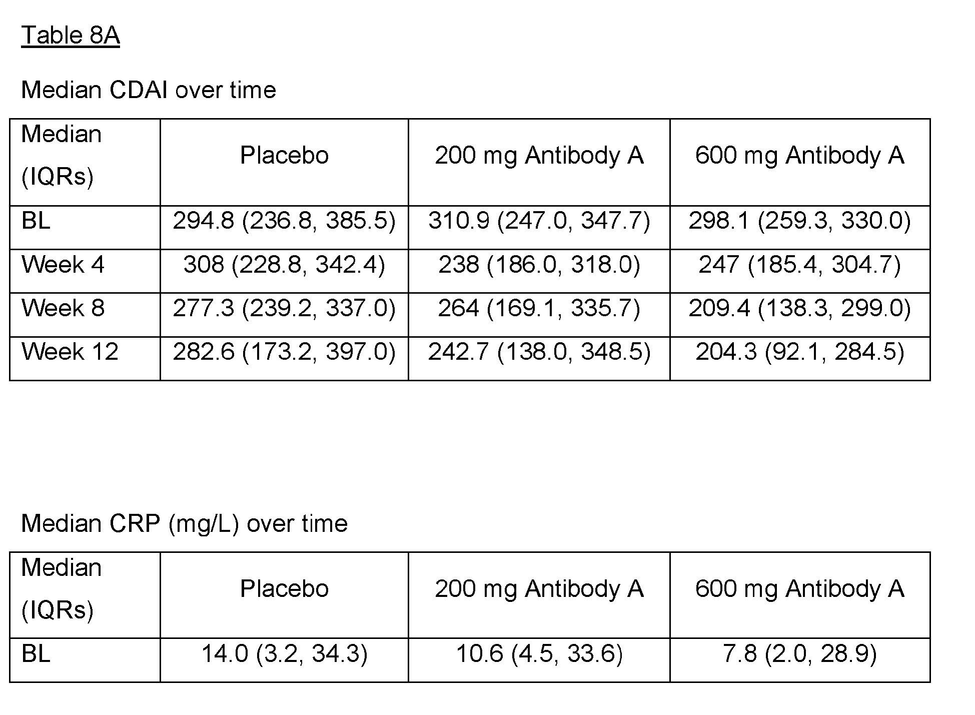

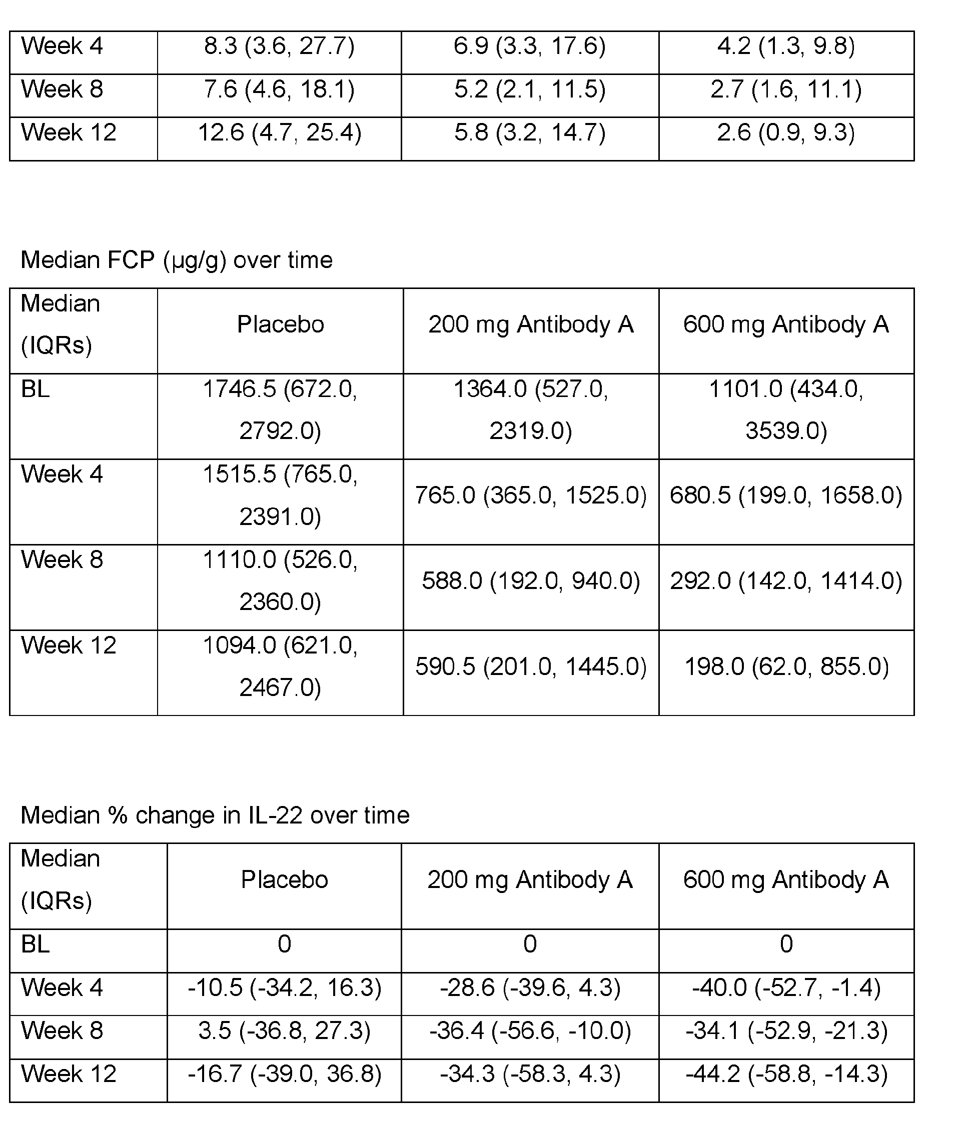

**Table 8B. PRO-2 response at Week 12.**

| PRO-2 response at Week 12 | | Placebo | Antibody A 200mg iv | Antibody A 600mg iv | Antibody A 200+600mg iv |
|---|---|---|---|---|---|
| Number of patients [N (%)] | | 39 (100.0) | 41 (100.0) | 41 (100.0) | 82 (100.0) |
| Number (%) satisfying PRO-2 response at Week 12 | | 11 (28.2) | 17 (41.5) | 19 (46.3) | 36 (43.9) |
| | 95% confidence interval[1] | (15.0, 44.9) | (26.3, 57.9) | (30.7, 62.6) | (33.0, 55.3) |
| Comparison vs. Placebo | | | | | |
| | Estimate of the difference | | 13.30 | 17.65 | 15.37 |
| | 95% confidence interval[2] | | (-6.9, 33.5) | (-2.6, 37.9) | (-2.0, 32.7) |
| | p-value[2] | | 0.1959 | 0.0873 | 0.0828 |
| Comparison vs. Antibody A 200mg iv | | | | | |
| | Estimate of the difference | | | 3.43 | |
| | 95% confidence interval[2] | | | (-17.2, 24.0) | |
| | p-value[2] | | | 0.7443 | |

| | | | | | |
|---|---|---|---|---|---|
| [1] Exact 95% CI by Clopper and Pearson [2] Statistics for the difference are calculated using the Cochran-Mantel-Haenszel risk difference stratified by TNF naive versus TNF experienced | | | | | |

**Table 8C. PRO-2 remission at Week 12.**

| PRO-2 remission at Week 12 | | Placebo | Antibody A 200mg iv | Antibody A 600mg iv | Antibody A 200+600mg iv |
|---|---|---|---|---|---|
| Number of patients [N (%)] | | 39 (100.0) | 41 (100.0) | 41 (100.0) | 82 (100.0) |
| Number (%) satisfying PRO-2 remission at Week 12 | | 6 (15.4) | 12 (29.3) | 15 (36.6) | 27 (32.9) |
| | 95% confidence interval[1] | (5.9, 30.5) | (16.1, 45.5) | (22.1, 53.1) | (22.9, 44.2) |
| Comparison vs. Placebo | | | | | |
| | Estimate of the difference | | 13.89 | 20.45 | 17.24 |
| | 95% confidence interval[2] | | (-4.0, 31.8) | (2.6, 38.3) | (2.3, 32.2) |
| | p-value[2] | | 0.1276 | 0.0245 | 0.0237 |
| Comparison vs. Antibody A 200mg iv | | | | | |
| | Estimate of the difference | | | 6.11 | |
| | 95% confidence interval[2] | | | (-13.6, 25.8) | |
| | p-value[2] | | | 0.5426 | |

| | | | | | |
|---|---|---|---|---|---|
| [1] Exact 95% CI by Clopper and Pearson [2] Statistics for the difference are calculated using the Cochran-Mantel-Haenszel risk difference stratified by TNF naive versus TNF experienced | | | | | |

### Molecular Profile

Colon and ileum tissue was collected at baseline and Week 12 from a subset of patients (63% 200 mg Antibody A, 66% 600 mg Antibody A, and 67% placebo). Significant reductions in the expression of selected genes associated with IL-23 immune-related pathways were observed in Antibody A-treated patients versus placebo (Table 9A).

**Table 9A. Selected Genes Decreased in the Colon by Antibody A at 12 Weeks PostTreatment, as Assessed by RNA Seq Analysis**

| **Differentially expressed gene by RNA seq analysis** | **Protein name** | **Antibody A treated (combined dose groups)** | |
|---|---|---|---|
| | | **Log₂ Fold Change (vs. baseline)** | **P-value** |
| **IL-23/IL-17 pathway related** | | | |
| *IL-23A* | Interleukin 23A | -3.07 | 0.0004 |
| *IL-26* | Interleukin 26 | -1.16 | 0.0024 |
| **S100 family of calcium-binding proteins** | | | |
| *S-100A8* | S100 calcium-binding protein A8 | -2.37 | 0.0023 |
| *S-100A9* | S100 calcium-binding protein A9 | -1.90 | 0.0034 |
| *S-100A12* | S100 calcium-binding protein A12 | -2.92 | 0.0018 |
| **Immune-related pathways** | | | |
| *IL-6* | Interleukin 6 | -3.07 | 0.0003 |
| *IL-8* | Interleukin 8 | -2.59 | 0.0029 |
| *IL-11* | Interleukin 11 | -2.66 | 0.0015 |
| *IL-18RAP* | Interleukin 18 RAP | -0.89 | 0.0026 |
| *IL-20RB* | Interleukin 20 RB | -0.39 | 0.0028 |

The molecular profile in the colon and/or ileum tissue was invertigated in a subset of anti-TNF experienced patients with CD, who received either 200 mg (n=26), 600 mg (n=27) Antibody A or placebo (n=26). From each patient, 6-9 biopsy samples were obtained from inflamed lesions in the colon or ileum at baseline and at 12 weeks posttreatment. Biopsy samples from ileum and from colon were separately analysed by transcriptome-wide RNA-Seq profiling. Univariate associations were assessed using linear regression. Effect size, p-values and FDR were calculated for significant genes. CDEIS response (>50% reduction from baseline) and CDEIS remission (≤4; for patients with isolated ileitis of ≤2) were evaluated at Week 12 by an independent blinded reviewer.

In an initial analysis, Antibody A treatment significantly decreased the expression of 1146 genes from baseline to Week 12 in the colon tissue of CD patients vs placebo (p<0.05). Of note, significant decreases in the expression of genes associated with the IL-23 pathway (*IL-23A*, *IL-26*, *IL-21R*, *IL-17A*, *STAT3*), innate immunity (*IL6*, *IL7*, *IL7R*, *IL8, ICAM1, IL1, IL11, IL13RA2, IL15RA, IL18R1, TNF),* tissue turnover (*S-100A8*, *A9*, *A12, MMP1, MMP3, MMP9, MMP12, ADAM8, ADAM12, ADAM33)* and solute carrier family (*SLC11A1*, *SLC1A3*, *SLC2A3*, *SLC2A6*, *SLC6A14*, *SLC7A11*, *SLC7A5*) were observed with Antibody A treatment. These overall changes in gene expression in the Antibody A-treated cohort reflected the molecular changes observed in patients achieving CDEIS response and remission at Week 12. A comparison of gene expression changes in the colon significantly modulated by Antibody A at Week 12 vs anti-TNF treatment at Week 14 in a published patient cohort highlighted larger decreases in suppression of pathways associated with epithelial biology (cell-cell adhesion, morphogenesis, intracellular signal transduction, second messenger signalling) following Antibody A treatment. In contrast, no significant changes were observed in the molecular profile in the ileum from patients treated with Antibody A vs placebo from baseline to Week 12.

A refinement of the analysis showed that Antibody A modulated 2744 genes expressed in the colon and 792 genes in the ileum with an overlap of 846 genes between colon and ileum. In the refined analysis, Antibody A treatment significantly decreased the expression of 1413 genes from baseline to Week 12 in the colon tissue of CD patients vs placebo (p<0.05). Antibody A treated patients who achieved endoscopic response or remission showed reduction in the expression of select genes in colon biopsies compared with patients who did not achieve endoscopic response or remission from baseline to Week 12. In patients with the most severe endoscopic lesions (deep ulcerations) at baseline, a larger proportion of Antibody A-treated patients achieved endoscopic response at Week 12 compared with placebo and these patients showed a transcriptomic pattern indicating reductions in the expression of key genes associated with active CD. Higher proportions of patients who had deep ulcerations at baseline in any segment of the colon achieved endoscopic response at Week 12 in the combined 200 mg and 600 mg Antibody A dose groups compared with patients with deep ulcerations in the ileum (Table 9B).

**Table 9B. Proportion of patients who achieved endoscopic response at Week 12 in patients with deep ulcerations at baseline in segments with biopsies.**

| **Location of deep ulceration, n (%)** | **Endoscopic response** | **Placebo** | **Antibody A** |
|---|---|---|---|
| **Colon*** | Yes | 1 (14.3) | 13 (44.8) |
| | No | 7 (85.7) | 16 (55.2) |
| **Ileum** | Yes | 0 | 5 (25) |
| | No | 5(100) | 15(75) |

| | | | |
|---|---|---|---|
| * includes rectum, sigmoid and left colon, right colon, transverse colon. CDEIS, Crohn's disease endoscopic index of severity. | | | |

### Reduction in expression of unique and common genes by Antibody A in colon vs ileum (baseline to week 12):

In an initial analysis, in the ileum, 173 unique genes were decreased by Antibody A (p<0.005). In the colon, 1418 unique genes were decreased by Antibody A (p<0.005). 630 common gene sets were decreased (p<0.005).

Among the common genes, which were decreased, were IL-23A, IL-22, IL-26, LCN2, IL21R, S-100A8, S-100A9, S-100A12, IL-8, TNF, MMP3, MMP1, TIMP1, CXCL10, ICAM-1, IFNg, JAK2, STAT3.

In a refined analysis, in the ileum, 298 unique genes were decreased by Antibody A (p<0.005). In the colon, 1413 unique genes were decreased by Antibody A (p<0.005). 467 common gene sets were decreased (p<0.005).

### The following pathways were modulated by treatment with Antibody A in the colon vs ileum at Week 12:

Differences between colon and ileum:
- stronger enrichment/ modulation for colon: Heme metabolism, Angiogenesis, Apical junction, fatty acid metabolism, hypoxia
- stronger enrichment/ modulation for ileum: Reactive oxygen species, apical surface, Peroxisome.

Commonalities between colon and ileum (strong enrichment/ modulation for both): Hypoxia, Interferon-a response, Apoptosis, Coagulation, IL-2 STAT5 signaling, Epithelial-mesenchymal transitioning, Complement, IL-6 JAK/STAT3 signaling, Interferon-y response, TNF-α signaling, Inflammatory response.

Treatment with Antibody A and not anti-TNF more strongly modulated select genes in pathways associated with second messenger mediated signaling, cellular defense response, cell-cell adhesion and cellular homeostasis.

A refined analysis showed that Antibody A treatment modulated genes associated with inflammatory response, several cytokine signalling pathways including IL-17, IL-6 JAK/STAT3, IFNγ, and IL-12 signalling, genes upregulated by KRAS activation and TNFα signalling via NFκB in both the colon and the ileum. Apart from IL-12 signaling, in all of these gene sets/ pathways more genes were differentially expressed in the colon compared to the ileum.

Other gene sets encoding IL-23 signalling, components of the complement system, angiogenesis, upregulated during transplant rejection (allograft-rejection) and epithelial-mesenchymal transition were only significantly enriched by genes deregulated in the colon based on the chosen cutoffs.

Antibody A more strongly decreased expression of genes associated with pathways linked to second messenger-mediated signalling, lymphocyte differentiation, lymphocyte activation, leucocyte activation, immune response and immune effector response compared with pathways associated with cell-cell adhesion, regulation of angiogenesis, anatomical structure and development in the colon.

### Assessment of fecal calprotectin and fecal lactoferrin and correlation with CDEIS

Calprotectin was assessed in fecal samples collected at baseline and Week 12 using the Bühlmann ELISA test (Schönenbuch, Switzerland) by Covance laboratories.

Lactoferrin was assessed in fecal samples collected at baseline and Week 12 using the IBD-SCAN^{®} (TechLab, Blacksburg, Virginia) by Covance laboratories. Spearman correlation coefficients were calculated for the correlations between the percent change from baseline in fecal calprotectin and fecal lactoferrin levels with change from baseline in CDEIS scores.

Significant reductions (baseline to Week 12) in faecal levels of calprotectin (74.4% vs 2.5%) and lactoferrin (69% vs 18.4%) proteins were observed in patients with CD treated with Antibody A compared with placebo. There were significant correlations between changes in faecal calprotectin (r=0.479, p=0.0059) or changes in lactoferrin levels (r=0.572, p=0.0007) and changes in CDEIS scores from baseline to Week 12 in patients treated with Antibody A. A correlation was observed between the expression of calprotectin at the protein level in faeces and the reduction in the expression of S-100A8 transcript in colon biopsies in patients treated with Antibody A (r=0.525, p=0.0238) and associated changes in the transcriptomic profile. Furthermore, mucosal transcription of S-100 A8 and A9 (calprotectin) were decreased in patients with reductions in protein levels of faecal biomarkers (calprotectin/lactoferrin).

### Assessment of miRNAs in colon and feces

Global transcriptome-wide sequencing of miRNA from 44 patients with colonic or ileocolonic CD was achieved using CleanTag^{™} Small RNA Library Prep Kit protocol (TriLink BioTechnologies, San Diego, CA, USA) according to the manufacturer's instructions and sequenced on the Illumina HiSeq 2000 (Illumina Inc., San Diego, CA). In addition, fecal miRNAs from 14 patients with matching colon biopsies were analyzed using a NanoString's human V3 CodeSet (based on miRBase v21) (NanoString Technologies, Seattle, WA, USA) that contains more than approximately 700 human miRNAs. In brief, total RNA was mixed with pairs of capture and reporter probes, hybridized on the nCounter Prep Station, and purified complexes were quantified on the nCounter Digital Analyzer and analyzed by nSolver software (v1.1; NanoString Technologies, Seattle, WA). Sequenced reads were adapter-trimmed and mapped to the human genome version hg19 using STAR aligner. Read counts were obtained using subread's featureCounts based on mirBase v19 annotation.

Data was normalized using the TMM method and the limma package was utilized to derive Log2 fold changes and corresponding False Discovery Rate (FDR) adjusted p-values. Briefly speaking, data was voom-transformed, correlations between baseline and Week 12 measurements per patient were estimated by the duplicateCorrelation function, a linear model was fit using the ImFit-function and finally moderated t-statistics were computed. For the NanoString data analysis, the background threshold for each lane was set to the mean count level of the negative controls plus two standard deviations. Next, the positive normalization factor was calculated as the mean of all positive controls divided by the mean of positive controls for the respective lane. Finally, miRNAs count levels were normalized to the geometric mean of the top 100 expressed miRNAs. The average of the geometric means of the top 100 expressed miRNAs for all lanes divided by the geometric mean of the respective lane is used as the normalization factor per lane.

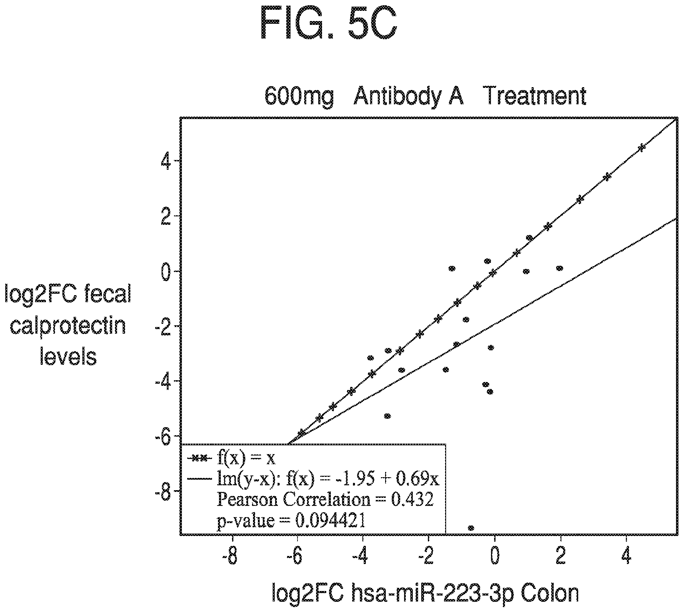

Small RNA sequencing of colon biopsies comparing baseline and Week 12 samples identified 18 significant differentially expressed miRNAs (FDR adjusted p<0.05) in patients treated with Antibody A compared with placebo (Figure 4). Of these, 13 miRNAs were downregulated and 5 upregulated by Antibody A. Comparing matching colon and faecal samples from 14 patients, a strong correlation was observed for miR-223-3p (Figures 5A and 5B). Downregulated levels of miR-223-3p at Week 12 were observed only in Antibody A treated patients. This result was not associated with endoscopic response or remission. Changes in levels of miR-223-3p correlated with changes in faecal calprotectin levels in the Antibody A dose group although the changes were not statistically significant (Figure 5C).

### Pharmacokinetic/Pharmacodynamic analysis

The relationship of Antibody A concentrations and CDAI or CDEIS categorical response at Week 12 indicates that the median (95% CI) CDAI and CDEIS response rates increase from 36% (23%, 51%) to 47% (33%, 62%) and 33% (21%, 49%) to 36% (23%, 53%) for the 200 mg to 600 mg median Week 12 concentrations, respectively (Table 10A).

Antibody A concentrations in plasma were sampled pre-dose and at Weeks 2, 4, 8, 12, 23, and 26, then every 8 weeks through Week 50 and at Weeks 52 and 65 (last visit). Concentrations were determined by a validated enzyme-linked immunosorbent assay. Pharmacokinetics were evaluated using a nonlinear mixed-effects population approach using NONMEM v7.3. The R software for statistical computing (package glm) was used for logistic regression analyses to examine the relationship of CDEIS and CDEI response as a dependent, categorical variable and Antibody A concentrations at Week 12 as an independent variable.

**Table 10A. Predicted CDAI and CDEIS Response Probability by Quantile of Antibody A**

| Treatment Group | Quantile of Antibody A concentration | Concentration at Week 12 | Predicted Response Probability | 95% Confidence Interval |
|---|---|---|---|---|
| Predicted CDAI response probability by quantile of Antibody A concentrations | | | | |
| Placebo | 0 | 0 | 0.33 | (0.18-0.51) |
| 200 mg | min | 1000 | 0.33 | (0.19-0.51) |
| | 0.25 | 5810 | 0.35 | (0.22-0.51) |
| | 0.5 | 8060 | 0.36 | (0.23-0.51) |
| | 0.75 | 12100 | 0.37 | (0.25-0.51) |
| | Max | 22600 | 0.41 | (0.31-0.53) |
| 600 mg | min | 2940 | 0.34 | (0.20-0.51) |
| | 0.25 | 22425 | 0.41 | (0.31-0.53) |
| | 0.5 | 36400 | 0.47 | (0.33-0.62) |
| | 0.75 | 44150 | 0.50 | (0.32-0.68) |
| | Max | 68900 | 0.60 | (0.29-0.85) |
| Predicted CDEIS response probability by quantile of Antibody A concentrations | | | | |
| Placebo | 0 | 0 | 0.33 | (0.17-0.52) |
| 200 mg | min | 1000 | 0.33 | (0.18-0.52) |
| | 0.25 | 5810 | 0.33 | (0.20-0.50) |
| | 0.5 | 8060 | 0.33 | (0.21-0.49) |
| | 0.75 | 12100 | 0.34 | (0.22-0.48) |
| | Max | 22600 | 0.35 | (0.25-0.47) |
| 600 mg | min | 2940 | 0.33 | (0.19-0.51) |
| | 0.25 | 22425 | 0.35 | (0.24-0.47) |
| | 0.5 | 36400 | 0.36 | (0.23-0.52) |
| | 0.75 | 44150 | 0.37 | (0.21-0.56) |
| | Max | 68900 | 0.40 | (0.14-0.73) |

**Table 10B. Predicted Clinical and Endoscopic Response and Remission Rates by Quartile of Antibody A Concentrations at Week 12.**

| **Treatment group** | **Antibody A concentration quartile** | **Median concentration at Week 12 (ng/ml)** | **Predicted response rate** | **95% confidence interval** |
|---|---|---|---|---|
| **Clinical response probability** | | | | |
| Placebo | 0 | 0 | 0.26 | (0·17-0·37) |
| 200 mg | Q1 | 4680 | 0·28 | (0·20-0·39) |
| | Q2 | 6470 | 0·29 | (0·21-0·39) |
| | Q3 | 9510 | 0·31 | (0·23-0·40) |
| | Q4 | 16200 | 0·35 | (0·26-0·44) |
| 600 mg | Q1 | 17100 | 0·35 | (0·27-0·45) |
| | Q2 | 27800 | 0·42 | (0·31-0·53) |
| | Q3 | 41200 | 0·50 | (0·34-0·67) |
| | Q4 | 48200 | 0·55 | (0·35-0·73) |
| **Clinical remission probability** | | | | |
| Placebo | 0 | 0 | 0·18 | (0·11-0·28) |
| 200 mg | Q1 | 4680 | 0·20 | (0·13-0·30) |
| | Q2 | 6470 | 0·21 | (0·14-0·30) |
| | Q3 | 9510 | 0·22 | (0·15-0·32) |
| | Q4 | 16200 | 0.26 | (0·19-0·35) |
| 600 mg | Q1 | 17100 | 0·27 | (0·19-0·36) |
| | Q2 | 27800 | 0·34 | (0·24-0·45) |
| | Q3 | 41200 | 0·43 | (0·28-0·60) |
| | Q4 | 48200 | 0·48 | (0·29-0·68) |
| **Endoscopic response probability** | | | | |
| Placebo | 0 | 0 | 0·23 | (0·14-0·35) |
| 200 mg | Q1 | 4690 | 0·24 | (0·16-0·35) |
| | Q2 | 6520 | 0·25 | (0·17-0·36) |
| | Q3 | 10300 | 0.26 | (0·18-0·36) |
| | Q4 | 17100 | 0·29 | (0·21-0·38) |
| 600 mg | Q1 | 17000 | 0·29 | (0·21-0·38) |
| | Q2 | 29700 | 0·33 | (0·23-0·46) |
| | Q3 | 41200 | 0·38 | (0·23-0·56) |
| | Q4 | 47700 | 0·41 | (0·23-0·62) |
| **Endoscopic remission probability** | | | | |
| Placebo | 0 | 0 | 0·09 | (0·04-0·18) |
| 200 mg | Q1 | 4690 | 0·10 | (0·05-0·19) |
| | Q2 | 6520 | 0·10 | (0·05-0·19) |
| | Q3 | 10300 | 0·11 | (0·06-0·19) |
| | Q4 | 17100 | 0·13 | (0·07-0·21) |
| 600 mg | Q1 | 17000 | 0·12 | (0·07-0·21) |
| | Q2 | 29700 | 0·16 | (0·09-0·27) |
| | Q3 | 41200 | 0·20 | (0·10-0·37) |
| | Q4 | 47700 | 0·23 | (0·10-0·45) |

Clinical response defined as either CDAI <150 or a CDAI reduction from baseline of ≥100 at Week 12. Clinical remission defined as CDAI <150 at Week 12. Endoscopic response defined as >50% CDEIS reduction from baseline. Endoscopic remission is defined as CDEIS ≤4 at Week 12 (≤2 for patients with initial isolated ileitis). Concentration quartiles correspond to min-25% (Q1), 25-50% (Q2), 50-75% (Q3), and 75%-max (Q4) quantile range.

**Table 11. Adverse Events Summary (On-Treatment at Week 12)**

| **Adverse events - n (%)** | | **Placebo (N=39)** | **Antibody A** | |
|---|---|---|---|---|
| | | | **200 mg (N=41)** | **600 mg (N=41)** |
| Any AE | | 32 (82) | 32 (78) | 31 (76) |
| Severe AEs | | 9 (23) | 6(15) | 3 (7) |
| Drug-related AEs | | 8 (21) | 10 (24) | 5 (12) |
| AEs leading to discontinuation | | 6 (15) | 5 (12) | 1 (2) |
| Serious AEs* | | 12 (31) | 9 (22) | 3 (7) |
| Persist or significant | | 0 | 1 (2) | 0 |
| disability/incapacity | | 10 (26) | 8 (20) | 2 (5) |
| Requiring or prolonging hospitalization | | 4 (10) | 1 (2) | 1 (2) |
| Other medically important serious event | | | | |
| Common AEs^{†} | | | | |
| | Nausea | 4 (10) | 8 (20) | 3 (7) |
| | Worsening of Crohn's disease | 6 (15) | 2 (5) | 0 |
| | Abdominal pain | 4 (10) | 6(15) | 3 (7) |
| | Arthralgia | 3 (8) | 6(15) | 6 (15) |
| | Anemia | 4 (10) | 0 | 2 (5) |
| | Headache | 4 (10) | 6(15) | 4 (10) |
| | Vomiting | 4 (10) | 3 (7) | 2 (5) |

Adverse events were coded using MedDRA v18.1. The severity of AEs was graded according to the RCTC v2.0.

*A serious AE was defined as any AE that results in death, is immediately life-threatening, results in persistent or significant disability/incapacity, requires or prolongs patient hospitalization, is a congenital anomaly/birth defect, or is an important medical event, based upon appropriate medical judgment that may jeopardize the patient and may require medical or surgical intervention.

^{†}Common AEs were reported in at least 10% of patients in any study arm.

AE, adverse event; RCTC, Rheumatology Common Toxicity Criteria

### Inflammatory Bowel Disease Questionnaire (IBDQ) assessment at Week 12

The IBDQ assessment indicated a reduced HRQoL of the randomized study population at baseline. Treatment with study drug resulted in a dose-dependent increase from baseline to Week 12 by 7.3, 21.7, and 34.7 points in the placebo, 200 mg, and 600 mg dose groups, respectively (Table 12).

**Table 12. Baseline and Change from Baseline at Week 12 in IBDQ Score.**

| | | **Placebo (N=39)** | **Antibody A** | |
|---|---|---|---|---|
| | | | **200 mg (N=41)** | **600 mg (N=41)** |
| Baseline | | | | |
| | N | 37 | 37 | 39 |
| | Mean | 119.3 | 110.2 | 111.5 |
| | SD | 34.9 | 30.1 | 27.8 |
| | Min | 53.0 | 53.0 | 52.0 |
| | Median | 125.0 | 108.0 | 112.0 |
| | Max | 191.0 | 167.0 | 169.0 |
| Change from baseline at Week 12 | | | | |
| | N | 32 | 35 | 37 |
| | Mean | 7.3 | 21.7 | 34.7 |
| | SD | 35.2 | 27.0 | 29.9 |
| | Min | -54.0 | -28.0 | -20.0 |
| | Median | -0.5 | 20.0 | 35.0 |
| | Max | 108 | 85.0 | 118.0 |

| | | | | |
|---|---|---|---|---|
| IBDQ, Inflammatory Bowel Disease Questionnaire; SD, standard deviation | | | | |

### Re-induction treatment

At Week 12, patients entered Period 2; here, patients in deep remission underwent washout and all other patients underwent open label intravenous re-induction therapy (600 mg Antibody A at Weeks 14, 18 and 22).

### Results:

Baseline demographics and disease characteristics were similar between study arms. Mean age was 38.1 years and median CDAI and CDEIS scores were 298 and 12; 94% of patients had previously received ≥1 TNF antagonists. At Week 12, clinical remission was achieved by 24.4% and 36.6% of patients with 200 and 600 mg Antibody A vs 15.4% for placebo (p=0.31 and p=0.025) and deep remission was achieved by 2.4% and 12.2% of patients with 200 and 600 mg Antibody A vs 0% for placebo (p=0.31 and p=0.016). In patients entering Period 2 without clinical remission, open label re-induction of placebo patients induced a rate of clinical remission similar to the 600 mg arm in the blinded Period 1, dose escalation from 200 to 600 mg induced a high clinical remission rate, and re-induction treatment in the 600 mg arm further increased the clinical remission rate in this group at Week 26 (Table 13). No patients with deep remission relapsed during the Antibody A washout phase to Week 26. Adverse events were similar between Antibody A and placebo with no dose-related increase in adverse events in Period 1. Antibody A was well tolerated in Period 2.

### Conclusions:

Re-induction therapy with 600 mg Antibody A was effective in capturing higher clinical remission rates at Week 26. Overall, Antibody A was well tolerated.

**Table 13. Period 2: Clinical remission at Week 26 vs Week 12**

| | | **Placebo** | **200 mg Antibody A** | **600 mg Antibody A** |
|---|---|---|---|---|
| Number of evaluable patients in Period 2 | | 30 | 34 | 34 |
| Patients in clinical remission at Week 12, N | | 6 | 8 | 9 |
| Patients in clinical remission at Week 26, n (%) | | | | |
| | Yes | 6 (100) | 6 (75) | 9 (100) |
| | No | 0 | 2 (25) | 0 |
| Patients not in clinical remission at Week 12, N | | 24 | 26 | 25 |
| Patients in clinical remission at Week 26, n (%) | | | | |
| | Yes | 12 (50) | 15 (58) | 8 (32) |
| | No | 12 (50) | 11 (42) | 17 (68) |
| The proportion of patients in clinical remission at Week 26 after open label intravenous re-induction therapy (600 mg Antibody A at Weeks 14, 18 and 22) in Period 2 are shown by original Period 1 treatment designation and prior Week 12 clinical remission status. All patients who were not in deep remission (clinical remission [CDAI <150] + endoscopic remission [CDEIS ≤4; for patients with initial isolated ileitis a CDEIS ≤2]) at Week 12 underwent Antibody A re-induction therapy. | | | | |

### Example 2: Pharmaceutical compositions

Examples of formulations suitable for an antibody of the present invention are shown below. Antibodies used in the formulations below are for example Antibody A, Antibody B, Antibody C or Antibody D.

### Formulation 1:

| **Components** | **Concentration [mmol/L]** | **Concentration [g/l]** | **Nominal Amount [mg/vial] V = 10.0 ml** |
|---|---|---|---|
| Antibody | | 10.0 | 100.0 |
| Succinic acid | 0.7 | 0.083 | 0.8 |
| Disodium succinate hexahydrate | 24.3 | 6.564 | 65.6 |
| Sodium chloride | 125 | 7.305 | 73.1 |
| Polysorbat 20 | 0.16 | 0.20 | 0.20 |
| Water for Injection | - | Ad 1L | Ad 1mL |

The pH of formulation 1 is typically in the range of pH 6.0 to 7.0, for example pH 6.5. This formulation is particularly suitable for intravenous administration.

Molecular weight (MW in g/mol) of used excipients: Disodium succinate hexahydrate = 270.14 g/mol; Succinic acid = 118.09 g/mol; Sodium chloride = 58.44 g/mol.

The osmolarity of the formulation is 300 +/- 30 mOsmol/kg, as determined using an Osmomat 030 (Gonotec GmbH, Berlin, Germany). The density at 20°C of the formulation is approximately 1.0089 g/cm³, as determined using a measuring unit DMA 4500 (Anton Paar GmbH, Ostfildern-Scharnhausen, Germany).

### Formulation 2:

| **Components** | **Concentration [mmol/L]** | **Concentration [g/l]** | **Nominal Amount [mg/syringe] V = 1.0 ml** |
|---|---|---|---|
| Antibody | 0.6 | 90.0 | 90.0 |
| Succinic acid | 0.5 | 0.059 | 0.059 |
| Disodium succinate hexahydrate | 3.9 | 1.054 | 1.054 |
| Sorbitol | 225 | 41.00 | 41.00 |
| Polysorbat 20 | 0.16 | 0.20 | 0.20 |
| Water for Injection | - | Ad 1L | Ad 1mL |

The pH of formulation 2 is typically in the range of pH 5.5 to 6.5, for example 5.5 to 6.1, for example the pH is 5.8. This formulation is particularly suitable for subcutaneous administration.

Molecular weight (MW in g/mol) of used excipients:
MW: Succinic acid (C₄H₆O₄)= 118.09 g/mol
MW: Disodium succinate hexahydrate (C₄O₄Na₂H₄ × 6H₂O) = 270.14 g/mol
MW: Sorbitol = 182.17 g/mol
MW: Polysorbate 20 = 1227.72 g/mol

The osmolarity of the formulation is 300 +/- 30 mOsmol/kg, as determined using an Osmomat 030 (Gonotec GmbH, Berlin, Germany). The density at 20°C of the formulation is approximately 1.040 g/cm³, as determined using a measuring unit DMA 4500 (Anton Paar GmbH, Ostfildern-Scharnhausen, Germany).

### Formulation 3:

| **Components** | **Concentration [mmol/L]** | **Concentration [g/l]** | **Nominal Amount [mg/syringe] V = 1.0 ml** |
|---|---|---|---|
| Antibody | 0.6 | 90.0 | 90.0 |
| Sorbitol | 240 | 43.733 | 43.733 |
| Polysorbat 20 | 0.16 | 0.20 | 0.20 |
| Water for Injection | - | Ad 1L | Ad 1mL |

The pH of formulation 3 is typically in the range of pH 5.5 to 6.5, for example 5.5 to 6.1, for example the pH is 5.8. This formulation is particularly suitable for subcutaneous administration.

Molecular weight (MW in g/mol) of used excipients:
MW: Sorbitol = 182.17 g/mol
MW: Polysorbate 20 = 1227.72 g/mol.

The osmolarity of the formulation is 300 +/- 30 mOsmol/kg, as determined using an Osmomat 030 (Gonotec GmbH, Berlin, Germany).

### Embodiments

1. A method for inducing remission of Crohn's Disease comprising administering to a patient an anti-IL-23A antibody, said method comprising:
   a) administering at least one induction dose of said anti-IL-23A antibody to the patient, wherein said induction dose comprises 750 mg of said anti-IL-23A antibody.
2. The method according to item 1, wherein 1, 2 or 3 induction doses are administered to the patient.
3. The method according to item 1 or 2, wherein 2 or 3 inductions doses are administered at 4 weeks intervals.
4. The method according to any one of items 1 to 3, wherein said induction dose is administered by intravenous infusion.
5. The method according to anyone of items 1 to 4, wherein said patient has a CDAI score of 220-450 before said administration in step a).
6. The method according to anyone of items 1 to 5, wherein said patient achieves a CDAI score of less than 150.
7. The method according to anyone of items 1 to 6, wherein said patient achieves a PRO-2 score equal to or less than 75.
8. The method according to any one of items 1 to 7, further comprising maintaining remission of Crohn's disease, said method further comprising:
   b) administering a first maintenance dose of said anti-IL-23A antibody to the patient after the last induction dose is administered; and
   c) administering at least one additional maintenance dose to the patient 4 to 12 weeks after said first maintenance dose is administered.
9. The method according to item 8, wherein said first maintenance dose is administered 2 to 8 weeks, for example 4 to 6 weeks, for example 2 weeks, 4 weeks, 6 weeks or 8 weeks, after the last induction dose is administered.
10. The method according to any one of items 8 or 9, wherein said at least one additional maintenance dose is administered to the patient 4, 8 or 12 weeks after said first maintenance dose is administered.
11. The method according to any one of items 8 to 10, wherein said first maintenance dose comprises 150 to 300 mg of said anti-IL-23A antibody.
12. The method according to any one of items 8 to 11, wherein said first maintenance dose comprises 150 mg, 225 mg or 300 mg of said anti-IL-23A antibody.
13. The method according to any one of items 8 to 12, wherein said first maintenance dose comprises 180 mg or 270 mg of said anti-IL-23A antibody.
14. The method according to any one of items 8 to 13, wherein said at least one additional maintenance dose comprises 150 to 300 mg of said anti-IL-23A antibody.
15. The method according to any one of items 8 to 14, wherein said at least one additional maintenance dose comprises 150 mg, 225 mg or 300 mg of said anti-IL-23A antibody.
16. The method according to any one of items 8 to 15, wherein said at least one additional maintenance dose comprises 180 mg or 270 mg of said anti-IL-23A antibody.
17. The method according to any one of items 8 to 16, wherein said first maintenance dose and said at least one additional maintenance dose comprise 150 to 300 mg of said anti-IL-23A antibody.
18. The method according to any one of items 8 to 17, wherein said first maintenance dose and said at least one additional maintenance dose comprise 150 mg, 225 mg or 300 mg of said anti-IL-23A antibody.
19. The method according to any one of items 8 to 18, wherein said first maintenance dose and said at least one additional maintenance dose comprise 180 mg or 270 mg of said anti-IL-23A antibody.
20. The method according to any one of items 8 to 19, wherein said maintenance dose is administered by subcutaneous injection.
21. The method according to anyone of items 8 to 20, wherein said patient maintains a CDAI score of less than 150.
22. The method according to anyone of items 8 to 21, wherein said patient maintains a PRO-2 score equal to or less than 75.
23. A method for treating Crohn's Disease comprising administering to a patient 750 mg of an anti-IL-23A antibody.
24. A method for treating Crohn's Disease comprising administering to a patient an anti-IL-23A antibody, said method comprising:
   a) administering at least one induction dose of said anti-IL-23A antibody to the patient, wherein said induction dose comprises 750 mg of said anti-IL-23A antibody.
25. The method according to item 24, further comprising:
   b) administering a first maintenance dose of said anti-IL-23A antibody to the patient after the last induction dose is administered; and
   c) administering at least one additional maintenance dose to the patient 4 to 12 weeks after said first maintenance dose is administered.
26. The method according to item 25, wherein said first maintenance dose comprises 150 to 300 mg of said anti-IL-23A antibody.
27. The method according to item 25 or 26, wherein said at least one additional maintenance dose comprises 150 to 300 mg of said anti-IL-23A antibody.
28. The method according to anyone of items 1 to 27, wherein said anti-IL-23A antibody is Antibody A, Antibody B, Antibody C or Antibody D.
29. The method according to anyone of items 1 to 28, wherein said method is for treating moderate to severe active Crohn's Disease.
30. A method for detecting the presence or absence of a beneficial response in a patient after administration of an IL-23A antagonist, comprising:
   a) obtaining a biological sample from the patient;
   b) measuring in said sample the level of expression of one or more genes, proteins or miRNAs;
   c) comparing the level in b) to the level of expression of the one or more genes, proteins or miRNAs in a control; and
   d) determining whether or not the difference in levels between the sample and the control reflects a beneficial response in the patient, wherein the one or more genes, proteins or miRNAs is one or more genes, proteins or miRNAs described herein.
31. The method according to item 30, wherein the patient suffers from Crohns' Disease.
32. The method according to item 30 or 31, wherein the biological sample is a colon tissue or a ileum tissue.
33. The method according to any one of items 30 to 32, wherein the biological sample is taken from the upper gatrointestinal (GI) tract, for example the stomach or the esophagus.
34. The method according to any one of items 30 to 33, wherein the IL-23A antagonist is an anti-IL-23A antibody or an antigen binding fragment thereof
35. The method according to any one of items 30 to 34, wherein the anti-IL-23A antibody is Antibody A, Antibody B, Antibody C or Antibody D.

## Claims

1. A method for detecting the presence or absence of a beneficial response in a patient after administration of an IL-23A antagonist, comprising:
a) obtaining a biological sample from the patient;
b) measuring in said sample the level of expression of one or more genes, proteins or miRNAs;
c) comparing the level in b) to the level of expression of the one or more genes, proteins or miRNAs in a control; and
d) determining whether or not the difference in levels between the sample and the control reflects a beneficial response in the patient, wherein the one or more genes, proteins or miRNAs is one or more genes, proteins or miRNAs described herein.

2. The method according to claim 1, wherein the patient suffers from Crohn's Disease.

3. The method according to claim 1 or 2, wherein the biological sample is a colon tissue or an ileum tissue.

4. The method according to any one of claims 1 to 3, wherein the biological sample is taken from the upper gastrointestinal (GI) tract, for example the stomach or the esophagus.

5. The method according to any one of claims 1 to 4, wherein the IL-23A antagonist is an anti-IL-23A antibody or an antigen binding fragment thereof

6. The method according to any one of claims 1 to 5, wherein the anti-IL-23A antibody is Antibody A, Antibody B, Antibody C or Antibody D.
